(19) Europäisches Patentamt
European Patent Office
Office européen des brevets

(11) **EP 2 391 643 B1**

(12) **EUROPÄISCHE PATENTSCHRIFT**

(45) Veröffentlichungstag und Bekanntmachung des Hinweises auf die Patenterteilung:
**24.08.2016 Patentblatt 2016/34**

(21) Anmeldenummer: **10701875.6**

(22) Anmeldetag: **01.02.2010**

(51) Int Cl.:
*C07K 14/435* (2006.01)     *C12N 15/00* (2006.01)
*C12N 15/62* (2006.01)

(86) Internationale Anmeldenummer:
**PCT/EP2010/051165**

(87) Internationale Veröffentlichungsnummer:
**WO 2010/086439 (05.08.2010 Gazette 2010/31)**

(54) **AUTOKATALYTISCHER PROTEINLINKER UND SEINE VERWENDUNG ZUR HERSTELLUNG VON FUSIONSPROTEINEN**

AUTOCATALYTIC PROTEIN LINKER AND USE THEREOF FOR PRODUCING FUSION PROTEINS

LIEUR PROTÉIQUE AUTOCATALYTIQUE ET SON UTILISATION POUR FABRIQUER DES PROTÉINES DE FUSION

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.02.2009 DE 102009008252**

(43) Veröffentlichungstag der Anmeldung:
**07.12.2011 Patentblatt 2011/49**

(73) Patentinhaber: **Technische Universität Dresden**
**01069 Dresden (DE)**

(72) Erfinder:
• **GÖTTFERT, Michael**
**01728 Bannewitz OT Hänichen (DE)**
• **ZEHNER, Susanne**
**01277 Dresden (DE)**
• **FRIEDRICH, Lars**
**80634 München (DE)**
• **WENZEL, Mandy**
**01217 Dresden (DE)**
• **SCHIRRMEISTER, Jana**
**01217 Dresden (DE)**

(74) Vertreter: **Kailuweit & Uhlemann Patentanwälte Partnerschaft mbB**
**Postfach 320 139**
**01013 Dresden (DE)**

(56) Entgegenhaltungen:
• **DATABASE UniProt [Online] 1. Juni 2001 (2001-06-01), "SubName: Full=Blr1806 protein; SubName: Full=ID205;" XP002576137 gefunden im EBI accession no. UNIPROT:Q9ANI0 Database accession no. Q9ANI0**
• **DATABASE UniProt [Online] 1. Juni 2003 (2003-06-01), "SubName: Full=Blr1649 protein;" XP002576138 gefunden im EBI accession no. UNIPROT:Q89TX4 Database accession no. Q89TX4**
• **DATABASE UniProt [Online] 1. Juli 2008 (2008-07-01), "SubName: Full=Putative uncharacterized protein;" XP002576139 gefunden im EBI accession no. UNIPROT:B2TCZ1 Database accession no. B2TCZ1**
• **DATABASE UniProt [Online] 30. Mai 2006 (2006-05-30), "SubName: Full=Putative uncharacterized protein;" XP002576140 gefunden im EBI accession no. UNIPROT:Q1NCW0 Database accession no. Q1NCW0**
• **DATABASE Geneseq [Online] 2. Juni 2005 (2005-06-02), "M. xanthus protein sequence, seq id 16066." XP002576142 gefunden im EBI accession no. GSP:ABM96867 Database accession no. ABM96867**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 391 643 B1

- **CHONG S ET AL: "Single-column purification of free recombinant proteins using a self-cleavable affinity tag derived from a protein splicing element" GENE, ELSEVIER, AMSTERDAM, NL LNKD- DOI:10.1016/S0378-1119(97)00105-4, Bd. 192, Nr. 2, 1. Januar 1997 (1997-01-01), Seiten 271-281, XP004081721 ISSN: 0378-1119**

- **SADILKOVA L ET AL: "Single-step affinity purification of recombinant proteins using a self-excising module from Neisseria meningitidis FrpC" PROTEIN SCIENCE 200810 US, Bd. 17, Nr. 10, Oktober 2008 (2008-10), Seiten 1834-1843, XP002576143**

**Beschreibung**

Die Erfindung betrifft die Verwendung eines autokatalytischen Proteinlinkers, Fusionsproteine, die diesen umfassen, einen Expressionsvektor, einen Kit zur Herstellung derartiger Fusionsproteine sowie deren Verwendung.

Die Erfindung findet Anwendung auf dem Bereich der Biochemie, der Biotechnologie und der Molekularbiologie, insbesondere bei der Proteinaufreinigung.

Mit der Sequenzierung kompletter Genome und der Annotation zahlreicher putativer Gensequenzen sieht sich die Wissenschaft vor die große Aufgabe gestellt, einer kaum überschaubaren Anzahl unbekannter Proteine eine Funktion zuzuordnen. Manchmal sind Vorhersagen über die Funktion eines unbekannten Proteins über Sequenzvergleiche mit bereits bekannten Proteinen möglich. Oftmals gibt es jedoch gar keine Hinweise auf die Funktion des putativen Genprodukts.

Um den hohen experimentellen Aufwand bei der Aufklärung der Funktion unbekannter Proteine zu verringern, hat die Molekularbiologie eine Vielzahl von Techniken entwickelt, mittels derer die Erforschung von unbekannten Proteinen vereinfacht und beschleunigt wird. Dabei spielen oft immunologische Methoden eine wichtige Rolle. Antikörper können spezifisch an Proteine binden und damit zur Aufreinigung und zur Detektion von Proteinen verwendet werden. Die Produktion eines proteinspezifischen Antikörpers erfordert jedoch einen hohen Arbeits- und Zeitaufwand und führt nicht in jedem Fall zum gewünschten Erfolg. Außerdem können Antikörper unterschiedlich stark affin zu den gewünschten Epitopen sein und manchmal auch Kreuzreaktivitäten zu unerwünschten Epitopen aufweisen.

Aus diesem Grund wird in der Molekularbiologie oft dem Einsatz von Fusionsproteinen der Vorzug gegeben.

Vor allem Fusionen eines zu exprimierenden Proteins (Zielprotein) mit Proteinmarkern oder Affinitätstags haben für die Molekularbiologie große Bedeutung erlangt, denn sie erlauben den spezifischen Nachweis und erleichtern die Aufreinigung der Zielproteine, ohne dass die Herstellung eines spezifischen Antikörpers gegen das betreffende Zielprotein nötig ist. Affinitätstags sind Proteine, Proteindomänen oder auch kurze Peptide, die spezifisch an einen bekannten Liganden binden. Bei diesen Liganden handelt es sich um organische Moleküle, Peptide, Proteine, Haptene oder auch Antikörper. Über die Bildung von Fusionsproteinen mit solchen Affinitätstags können Proteine, die selbst keinen bzw. keinen bekannten Liganden binden, einfach und bequem aufgereinigt werden. In der Affinitätschromatographie wird das gewünschte Zielprotein mit einem solchen Affinitätstag fusioniert und über die Bindung des Affinitätstags zu dem an einer stationären Phase immobilisierten Liganden schnell und spezifisch aus einem komplexen Gemisch angereichert.

Aber auch in anderen Bereichen der Molekularbiologie erweisen sich Fusionsproteine als nützlich. Durch das Verfahren der Immunpräzipitation kann ein Fusionsprotein durch die Bindung eines Antikörpers an den Affinitätstag gezielt aus einem Proteingemisch ausgefällt werden. Der Nachweis von Fusionsproteinen im Immunoblot erfolgt durch die Detektion mit einem spezifischen Antikörper gegen den Affinitätstag schnell und hochspezifisch.

In zytologischen Präparaten ist ein Fusionsprotein, bestehend aus dem Zielprotein und einem fluoreszierenden Fusionspartner, leicht durch Fluoreszenzmikroskopie nachweisbar. Fusionsproteine mit einem Affinitätstag können durch immunhistochemische Verfahren detektiert werden.

Viele weitere biochemische und molekularbiologische Verfahren beruhen auf der Synthese von Fusionsproteinen, wie beispielsweise der Yeast-Two-Hybrid-Assay und die Coimmunopräzipitation. Für all diese Verfahren wurden verschiedene Fusionspartner entwickelt und beschrieben, von denen sich viele in der Molekularbiologie mittlerweile fest etabliert haben. Beispiele für gebräuchliche Fusionen mit kompletten Proteinen oder Proteindomänen sind ß-Galactosidase, Protein A, Avidin, Streptavidin, Ubiquitin, GFP (green fluorescent protein), GST (glutathione S transferase), MBP (maltose binding protein), Calmodulin oder Thioredoxin.

Weil große Proteine in Fusionsproteinen jedoch unter Umständen die Funktionalität ihres Fusionspartners beeinträchtigen können, wurde mittlerweile auch eine große Zahl kurzer Peptide bzw. Peptid-Tags entwickelt, die für die Herstellung von Fusionsproteinen eingesetzt werden. Viele von diesen werden von spezifischen Antikörpern erkannt (HA-Tag, Myc-Tag, Flag-Tag,...). Andere weisen zusätzlich spezielle Bindungseigenschaften auf, die für die Aufreinigung des Fusionsproteins verwendet werden können, wie beispielsweise die Bindung an $Ni^{2+}$- oder $Zn^{2+}$-Ionen durch den His-Tag.

Selbst kurze Fusionspartner können jedoch im ungünstigsten Fall die Funktionalität des gewünschten Zielproteins beeinflussen, weshalb der Fusionspartner nach der Aufreinigung des Fusionsproteins oft vom Zielprotein abgetrennt werden soll. Zur Abtrennung des Fusionspartners wird in den meisten Fällen die enzymatische Spaltung zwischen Fusionspartner und Zielprotein, gefolgt von der Abtrennung des abgespaltenen Fusionspartners und des proteolysierenden Enzyms, eingesetzt. Der Einsatz verschiedener Endoproteasen (z.B. Thrombin, TEV Protease) ist dabei am weitesten verbreitet. Diese Enzyme erkennen eine spezifische Sequenz, die zwischen Fusionspartner und Zielprotein kloniert wurde. Eine Voraussetzung für eine erfolgreiche Spaltung ist die Zugänglichkeit dieser Schnittstelle für die Protease. Die Sequenz des Zielproteins darf zusätzlich keine Erkennungssequenz für diese Protease enthalten. Benachbart zur Spaltstelle kann das Zielprotein nach der Spaltung vom Affinitätstag zusätzliche Aminosäurereste tragen. Das kann den Einsatz der Enzyme beschränken (Arnau et al. 2006).

Bei der Aufreinigung des Zielproteins müssen das proteolytische Enzym und der Fusionspartner nach der Spaltung abgetrennt werden. Damit wird ein zusätzlicher Reinigungsschritt notwendig, bei dem häufig modifizierte Proteasen (z.B. biotinyliertes Thrombin, GST-verknüpfte PreScission-Protease) zum Einsatz kommen, die durch Affinitätschromatographie abtrennbar sind. Für die Abtrennung des Fusionspartners ohne am Zielprotein verbleibende Aminosäurereste können Enterokinase oder die Faktor Xa Protease verwendet werden.

Die allgemeinen Nachteile beim Einsatz proteolytischer Enzyme sind die große benötigte Menge an Enzym, eine potenzielle unspezifische Spaltung oder Degradation und eine relativ lange Inkubationszeit zum Erreichen einer guten Ausbeute an Zielprotein. Eine andere Methode zur Entfernung kleiner Fusionspartner ist daher z.B. der Einsatz von Exopeptidasen. Diese Enzyme spalten Aminosäuren oder kurze Peptide (zumeist Dipeptide) von einem Ende des Proteins ab, bis zum Erreichen eines Stoppsignals (Pedersen et al. 1999, Kenig et al. 2006, Arnau et al. 2006). Diese Technik erlaubt das Entfernen aller nicht-natürlichen Aminosäurereste vom Zielprotein. Aber auch hier ist die anschließende Abtrennung des Enzyms für die Reinigung des Zielproteins notwendig.

Neben diesem Schwachpunkt hat die Zugabe von Proteasen oder Peptidasen weitere Nachteile: Der Einsatz dieser Enzyme ist in der Regel mit einem hohen Kostenaufwand verbunden, der vor allem bei der großtechnischen Herstellung von rekombinanten Proteinen ins Gewicht fällt. Abgesehen davon kann auch nicht ausgeschlossen werden, dass die von Proteasen und Peptidasen erkannte Sequenz auch innerhalb des Zielproteins vorhanden ist. Es muss daher immer überprüft werden, ob das gewünschte System auch für das Zielprotein geeignet ist.

Daher ist es erstrebenswert Systeme bereitzustellen, die keine Zugabe von Proteasen oder Peptidasen erfordern. Ein möglicher Lösungsansatz basiert auf Fusionsproteinen, die selbstspaltende oder autoproteolytische Sequenzen enthalten.

Einige selbstspaltende Proteasen, wie beispielsweise der katalytische Kern von Sortase A oder Sortase B aus *Staphylococcus aureus* oder ein rekombinant verändertes Subtilisin, wurden mit Sequenzen von Affinitätstags sowie einem Zielprotein zu einem Fusionsprotein verknüpft (Mao 2004, Ruan et al. 2004). Durch den Zusatz eines niedermolekularen Cofaktors wird die Aktivität der Protease induziert. Die Spaltung des Zielproteins von der selbstspaltenden Protease und dem Affinitätstag kann erfolgen, während das Fusionsprotein an einer Affinitätsmatrix gebunden ist. Das Zielprotein wird eluiert und der Affinitätstag sowie das selbstspaltende Enzym verbleiben an der Matrix. Eine zusätzliche Abtrennung des Enzyms entfällt. Häufig beobachtete Nachteile dieser Methode ist die geringe Ausbeute an Zielprotein bzw. die teilweise Degradation durch Restaktivität des Enzyms in der Zelle (Mao 2004).

Die Entwicklung einer Ein-Stufen-Reinigung durch den Einsatz von Protein-Spleißen wurde ebenfalls beschrieben. Zu dieser Gruppe gehören beispielsweise Fusionsproteine, die über ein sogenanntes Intein mit einem Affinitätstag verbunden sind. Inteine verfügen über selbstspaltende Aktivität, d.h. sie können Abschnitte ihrer Proteinsequenz autokatalytisch aus sich selbst herausschneiden. Dieser Prozess wird als "Protein-Spleißen" bezeichnet. Dabei binden die Fusionsproteine über den Affinitätstag an eine Affinitätsmatrix. Die Spleißaktivität des Inteins wird dann auf der Säule durch Zugabe von Reduktionsmitteln wie Dithiothreitol (DTT) induziert. Dabei ist die Peptidbindung zwischen Intein und dem Affinitätstag vorteilhaft so modifiziert, dass sie durch das Intein nicht gespalten wird. Das gewünschte Zielprotein wird dann durch die Inteinaktivität aus dem Fusionsprotein freigesetzt und kann dann ohne den Fusionspartner, bestehend aus Intein und Affinitätstag, eluiert werden. Dabei wirkt sich der relativ große Fusionspartner (~55 kDa) etwas nachteilig aus (Chong et al. 1997, Zhang et al. 2001).

Eine neue Methode zur Abspaltung eines Affinitätstags wurde beschrieben, bei dem das Protein FrpC, das einen nicht-enzymatischen Spaltprozess durchläuft, als Proteinlinker verwendet wird (Sadilková et al. 2008). Die Spaltung beruht auf einer labilen Peptidbindung zwischen Aspartat und Prolin und kann durch Zugabe von Calcium induziert werden. Das selbstspaltende Proteinmodul wird zwischen Affinitätstag und Zielprotein kloniert. Während der Reinigung wird das Zielprotein durch Zugabe von Calcium-Ionen vom Affinitätstag getrennt.

Nachteilig wird im Verfahren von Sadilková et al. (2008) die Bildung von höhermolekularen Vernetzungsprodukten beobachtet. Durch den Zusatz von Thiolverbindungen (Cystein, DTT, Mercaptoethanol) kann die inter- und intramolekulare Vernetzung im Verfahren von Sadilková et al. (2008) unterdrückt werden. Verschiedene Zielproteine könnten jedoch empfindlich auf solche Verbindungen reagieren.

Aufgabe der Erfindung ist daher, weitere Proteinlinker zur Verfügung zu stellen, die ohne die Zugabe einer Protease und auch unter nicht-reduzierenden Bedingungen ohne Bildung von unerwünschten Vernetzungsprodukten gespalten werden.

Diese Aufgabe wird gemäß Anspruch 1 gelöst durch die Verwendung eines autoproteolytischen Proteinlinkers als Teil eines Fusionsproteins. Dieser Proteinlinker umfasst eine DUF 1521-Domäne oder eine Teilsequenz davon. Die DUF 1521-Domäne ist ausgewählt aus den Sequenzen mit der SEQ ID No. 1 bis 8 oder einer homologen Sequenz mit wenigstens 70% Sequenzidentität (bevorzugt 80 %, besonders bevorzugt 90 %) zu mindestens einer dieser Sequenzen oder einer Teilsequenz davon. Eine Teilsequenz der DUF 1521-Domäne umfasst die für die autoproteolytische Aktivität benötigten Abschnitte der DUF 1521-Domäne sowie die Spaltstelle und umfasst vorzugsweise 140 AS, bevorzugt 100 AS, besonders bevorzugt 80 AS der Aminosäuresequenz der DUF 1521-Domäne. Im Fall der SEQ ID. No. 8 umfasst eine Teilsequenz vorzugsweise 100 AS, besonders bevorzugt 80 AS der Aminosäuresequenz der DUF 1521-Domäne. Das Fusionsprotein umfasst neben dem autoproteolytischen Proteinlinker mindestens ein weiteres Protein oder Peptid.

Die autoproteolytische Spaltung des erfindungsgemäß verwendeten Proteinlinkers wird durch die Zugabe von $Ca^{2+}$-Ionen induziert. Vorteilhaft ist die erfindungsgemäße Induktion der Spaltung durch $Ca^{2+}$-Ionen sehr spezifisch und kann durch andere zweiwertige Metallionen nicht bewirkt werden. Dies ermöglicht eine hohe Flexibilität bei der Auswahl der Reaktionsbedingungen.

Die $Ca^{2+}$-Ionen werden abhängig von der eingesetzten Proteinmenge vorzugsweise in einem zwischen 0,1fachem und 10000fachem, bevorzugt 1fachem bis 1000fachem, besonders bevorzugt 10fachem bis 100fachem, molaren Verhältnis an Ionen gegenüber dem zu spaltenden Protein eingesetzt.

Die Konzentration der $Ca^{2+}$-Lösung beträgt dabei vorzugsweise zwischen 100 nmol/l und 500 mmol/l, bevorzugt zwischen 1 mmol/l und 100 mmol/l, besonders bevorzugt über 3 mmol/l.

Die DUF 1521-Domäne ist eine bevorzugt ca. 170 AS lange, konservierte Proteindomäne bisher unbekannter Funktion, die in den Proteinen Blr1806 (auch NopE1 genannt) und Blr1649 (auch NopE2 genannt) von *Bradyrhizobium japonicum* gefunden wurde. Diese beiden Proteine enthalten jeweils zwei solcher DUF 1521-Domänen. Jede DUF 1521-Domäne verfügt über autokatalytische Aktivität, die durch die Anwesenheit von $Ca^{2+}$-Ionen induziert wird, und über eine spezifische Spaltstelle mit einer Sequenz DP oder EP. Die autokatalytische Spaltung erfolgt zwischen den Aminosäuren D (Aspartat) und P (Prolin), die den Aminosäuren D147 und P148, sowie D359 und P360 in dem Protein Blr1806 der Spezies *Bradyrhizobium japonicum* entsprechen, wobei die Aminosäure D auch gegen die verwandte Aminosäure E (Glutamat) ausgetauscht sein kann.

Erfindungsgemäß umfasst der autoproteolytische Proteinlinker eine DUF 1521-Domäne oder eine Teilsequenz davon, welche über die autokatalytische Aktivität und eine interne Proteaseschnittstelle verfügen. Dessen Aminosäuresequenz ist ausgewählt aus den Sequenzen gemäß SEQ ID No.1 bis 8 oder einer homologen Sequenz, die mindestens zu einer dieser Sequenzen 70 %, bevorzugt 80 %, besonders bevorzugt 90 % Sequenzidentität aufweist.

Die N-terminal gelegene DUF 1521-Domäne des Proteins Blr1806 von *Bradyrhizobium japonicum* besitzt die Sequenz SEQ ID-No. 1:

```
WTHEVKDGKATINLGDKYTITANEKDGTWTVRNNQTGHVTVIHGDPHVDADGDGKDDFDFK
KDMTFQLDDGTKITVNNVDYGNGQAISSKLTITNGHNAIVVEGLGDDKDGKNNLRVTQSNA
GMTLDELTSDGAQTIHESGQGWVDGAGRAVNQASIDDGEQGRGPGNYQ
```

Die C-terminal gelegene DUF 1521-Domäne des Proteins Blr1806 von *Bradyrhizobium japonicum* besitzt die Sequenz SEQ ID-No. 2:

```
WSHEVRDGKAEIKLGDKYSILVDENDGTVLIRNSQTGKITSIKGDPHVDADGDGKVDFDFK
KNMTFQLDDGTKITVDTVDIGKGKTMASKLTITNGDNAMVVEGLGDRFDGKNNLKVTQSNA
GRTLDQLTSDGAQTIYEQPGSGWVDRSGRQVNQEIIDSNENPGTTSDA
```

Die N-terminal gelegene DUF 1521-Domäne des Proteins Blr1649 von *Bradyrhizobium japonicum* besitzt die Sequenz SEQ ID-No. 3:

```
WTHEVKDGKATINLGDKYTITANEKDGTWTVRNNQTGHVTKIHGDPHVDANGDGKDDFDFK
KDMTFQLDDGTKITVNNVDYGNGETISSRLTITNGHNAMVVEGLGDDKDGKNNLRVTQSNA
GMTLDELTPDGAQTIHESGQGWVDGAGGVVNQASIDAGEQGRAPGNYQ
```

Die C-terminal gelegene DUF 1521-Domäne des Proteins Blr1649 von *Bradyrhizobium japonicum* besitzt die Sequenz SEQ ID-No. 4:

```
WSHEVKDGKATINLGDKYTITADEKDGTWTVRNNQTGHVSRIHGDPHVDANGDGKDDFDFK
KGMTFQLDDGTKITVDTVNYGKGKTISSKLTITNGDNAMVVEGLGDDKDGKNNLKVTQSNA
GRTLDQLTSDGAQTIHETNQGWVDNSGRKVTQSVINHNENPDAPPDFK
```

Eine weitere DUF 1521-Domäne aus *Burkholderia phytofirmans* besitzt die Sequenz SEQ ID-No. 5:

WSNTQVNDNKSTIDLGNYKLDLNKKDSSMLLTDKKSGETTKVWGDPHIDSNGTSNMFNGPL
SLNLSDGTKITVGTQGKGNVSYADKLTITKGNDAYLVNGLSEKDSNPLTVQHAGNGRQLDA
MTPDGYSLVANQNGKGWIDPQTGHAPTAADFKKH

Eine weitere DUF 1521-Domäne aus *Sphingomonas* sp. SKA58 besitzt die Sequenz SEQ ID No. 6:

SASMEGESQASIDLGDGYSLELDERNSEIRIYNASTGETTRVWGDPHVDIDGKHAFDFWGT
TSFELENGTKITIDTEQYAANPNEYLASKLTITKGDQAIVVDGISQNQIGDLSITQSNNGQ
MLDMLTRDGFVLQENATGAGWRSELTGDIATQADLNLTKVGAIYGPGSE

Eine weitere DUF 1521-Domäne aus *Vibrio coralliilyticus* BAA450 besitzt die Sequenz SEQ ID No. 7:

SYEPSSGKATLENDRYTINIDESSSEIEVIDKQNPEDSFRIYGDPHFDIGNDGDTDFDFKK
DMSIELDDGTKLHIHTTPTSNGETLATSLAIEEPDGSGWYIEGIDSDQKGDLEVKEYNNIN
YSGGTVNDDAALELQVRDGNYFLNSD

Eine weitere DUF 1521-Domäne aus *Myxococcus xanthus* DK1622 besitzt die Sequenz SEQ ID No. 8:

NSLKVEEGTGKITTPGGYTIEQLGQFEWRVTGPDGKNTRVWGDPHVDESDGGKFDFKRDTS
FVLGDGTRINCSCKPWGNGMTVTGQLDVVYGDSHVRVTDIDKGKGKVGQVTNKGMD

Der erfindungsgemäß verwendete autoproteolytische Proteinlinker enthält folgende Sequenzmotive (bevorzugt in Richtung vomN-Terminus zum C-Terminus):

a.) die Spaltstelle mit der Sequenz DP oder EP, bevorzugt eingebettet in die Sequenz GDPH oder GEPH,

b.) bevorzugt ein EF-Hand-ähnliches Motiv, bevorzugt mit der Sequenz (V/I/F)D(A/S/I/E)(D/N/G/S)(G/N)D(G/D)(K/T/D/G)(D/V/S/H/T/G)(D/N/A/K)(F/M) DF(K/N/W)(K/G/R) (SEQ ID No. 9) in einer Entfernung von ca. 3 bis 10 AS C-terminal von der Spaltstelle

c.) bevorzugt mindestens eines der konservierten Motive:

(T/S)(F/L/I)(Q/N/E/V)L(D/S/E/G)(D/N)GT(K/R)(I/L)(T/H/N)(V/I/C) (SEQ ID No. 10); (K/R/S/Q)L(T/A/D)(I/V)(T/E/V) (SEQ ID No. 11); (V/I)(E/N/D/T)(G/D)(L/I) (G/S/D) (SEQ ID No. 12) und (T/N)(S/P/R/Y)(D/S)G(A/Y/F/G)(Q/S/V/T) (SEQ ID No. 13).

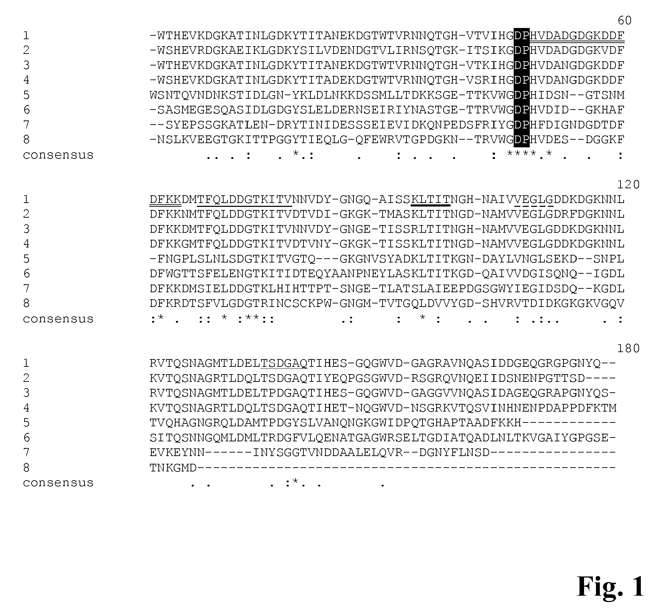

Figur 1 zeigt die entsprechenden Sequenzmotive in einem Alignment der Sequenzen SEQ ID No. 1 bis 8. Die Sequenzmotive sind in der Konsensussequenz und beispielhaft in der Sequenz SEQ ID No. 1 wie folgt markiert: Die Spaltstelle ist mit weißen Buchstaben auf dunklem Hintergrund gekennzeichnet und das EF-Hand-ähnliche Motiv ist doppelt unterstrichen. Die konservierten Motive (T/S)(F/L/I)(Q/N/E/V)L(D/S/E/G) (D/N)GT(K/R)(I/L)(T/H/N)(V/I/C) (SEQ ID No. 10, einfach unterstrichen), (K/R/S/Q)L(T/A/D)(I/V)(T/E/V) (SEQ ID No. 11, fett unterstrichen), (V/I)(E/N/D/T) (G/D)(L/I) (SEQ ID No. 12, gestrichelt unterstrichen) und (T/N)(S/P/R/Y)(D/S) G(A/Y/F/G)(Q/S/V/T) (SEQ ID No. 13, mit einer Wellenlinie unterstrichen) sind ebenfalls kenntlich gemacht.

N-terminal von der Spaltstelle enthält der autoproteolytische Proteinlinker vorzugsweise mindestens 36 Aminosäuren der DUF 1521-Domäne. C-terminal von der Spaltstelle enthält der autoproteolytische Proteinlinker bevorzugt mindestens 20 Aminosäuren, besonders bevorzugt mindestens 90 Aminosäuren der DUF 1521-Domäne.

Gegenstand der Erfindung ist auch der autoproteolytische Proteinlinker selbst, d.h. ein Peptid, das wie oben definiert eine DUF 1521-Domäne oder eine Teilsequenz davon umfasst.

Die Aufgabe wird weiterhin gelöst durch ein Fusionsprotein gemäß Anspruch 2, welches

a.) mindestens einen erfindungsgemäß verwendeten autoproteolytischen Proteinlinker und

b.) mindestens ein weiteres zu exprimierendes Protein als Fusionspartner

umfasst.

Dabei ist der autokatalytische Proteinlinker bevorzugt entweder über seinen N-Terminus durch eine Amidbindung mit dem C-Terminus des Fusionspartners oder über seinen C-Terminus durch eine Amidbindung mit dem N-Terminus des Fusionspartners verknüpft. Zusätzlich ist der autokatalytische Proteinlinker gegebenenfalls auch über seinen N-Terminus mit dem C-Terminus eines Fusionspartners und über seinem C-Terminus mit dem N-Terminus eines anderen Fusionspartners verbunden. In einer Ausgestaltungsform der Erfindung sind innerhalb eines Fusionsproteins mehrere Proteine oder Peptide über mehr als einen autokatalytischen Proteinlinker verknüpft.

Bevorzugt ist ein Fusionspartner ein Affinitätstag, d.h. ein Protein oder ein Peptid, das einen Liganden spezifisch binden kann und/oder für das ein spezifisch bindender Antikörper vorhanden ist. Der Affinitätstag ist bevorzugt ausgewählt aus Protein A, Avidin, Streptavidin, GST (glutathione S transferase), MBP (maltose binding protein), CBP (Chitin binding Protein), His-Tag, HA-Tag, Myc-Tag, Flag-Tag, Strep-Tag, Halo-Tag, HQ-Tag, CD-Tag, S-Tag, Avi-Tag, TAP-Tag, SF-Tag, Calmodulin oder Thioredoxin. Besonders bevorzugt ist der autokatalytische Proteinlinker mit zwei Fusionspartnern verknüpft, von denen einer ein Protein, vorzugsweise ein zu exprimierendes Protein (Zielprotein), und der andere ein Affinitätstag ist. Ein solches Fusionsprotein ist vorteilhaft geeignet für die Aufreinigung des Zielproteins über Affinitätschromatographie.

Ebenfalls bevorzugt ist ein Fusionsprotein, in dem der autokatalytische Proteinlinker ein Protein mit einem Markerprotein verknüpft, das bevorzugt eine nachweisbare Eigenschaft oder Aktivität aufweist, wie z. B. eine enzymatische Aktivität, wie ß-Galactosidase, Luciferase oder Alkalische Phosphatase, oder Fluoreszenz, wie GFP (green fluorescent protein), YFP, CFP, DsRed oder Tetracystein-Tags.

Weitere bevorzugte Fusionspartner sind Peptide oder Proteine, die Signalsequenzen umfassen, die für die Lokalisation des Fusionsproteins innerhalb der Zelle oder für seine Sekretion aus der Zelle verantwortlich sind. Eine solche Signalsequenz ist bevorzugt verantwortlich für die Sekretion des Fusionsproteins aus der Wirtszelle in das Medium bzw. bei der Verwendung von gram-negativen Bakterien als Wirtszelle auch in den periplasmatischen Raum, wodurch vorteilhaft der Aufschluss der Wirtszelle vermieden und die Aufreinigung des Fusionsproteins vereinfacht wird.

In einer Ausführungsform ist der autokatalytische Proteinlinker nur mit einem zu exprimierenden Protein (Zielprotein) verknüpft. In diesem Fall kann der autokatalytische Proteinlinker selbst als Affinitätstag dienen. Durch einen Antikörper oder ein Aptamer, der gegen den gesamten oder einen Teil des autokatalytischen Proteinlinkers gerichtet ist, wird vorteilhaft die affinitätschromatographische Aufreinigung des Fusionsproteins ermöglicht. Nach der Spaltung des autokatalytischen Proteinlinkers wird das Zielprotein freigesetzt. Der Begriff Antikörper im Sinne der vorliegenden Erfindung umfasst dabei neben monoklonalen und polyklonalen Antikörpern auch rekombinante Antikörper und Fragmente, wie z. B. scFv (single-chain-Fragmente) und Fab-Fragmente. Bevorzugt trägt der Antikörper ein Markermolekül, wie z. B. Biotin, Digoxigenin oder einen Fluoreszenzfarbstoff.

Eine Gensequenz gemäß Anspruch 7 kodierend für ein erfindungsgemäßes Fusionsprotein ist ebenfalls Bestandteil der Erfindung.

Das erfindungsgemäße Fusionsprotein wird bevorzugt in einer Wirtszelle rekombinant exprimiert. Daher ist auch ein Expressionsvektor gemäß Anspruch 9 Bestandteil der Erfindung. Der Expressionsvektor ist bevorzugt ein Plasmid, ein künstliches Chromosom oder auch ein Viruspartikel oder ein anderer Expressionsvektor, der eine Expressionskassette enthält, die stabil in das Genom des Wirts (Wirtszelle oder -organismus) eingebaut wird. Der Expressionsvektor enthält beispielsweise ein Affinitätstag oder ein Markerprotein.

Ein solcher Expressionsvektor erlaubt das Einfügen der Nukleinsäure, die für den oder die Fusionspartner kodiert, in den Expressionsvektor. Die kodierende Nukleinsäure wird über die Klonierungsstelle so in den Expressionsvektor integriert, dass die kodierende Sequenz einen fortlaufenden Leserahmen mit der für den erfindungsgemäß verwendeten autoproteolytischen Proteinlinker kodierenden Nukleinsäure bildet.

Das Einbringen des Expressionsvektors in die Wirtszelle erfolgt mittels dem Fachmann geläufiger chemischer, physikalischer und biologischer Techniken, wie beispielsweise Elektroporation, Calcium-Phosphat-Präzipitation, Lipofektion, Mikroinjektion und Transfektion.

Eine Wirtszelle ist eine natürlich vorkommende Zelle oder eine transformierte oder genetisch veränderte Zelllinie oder ein (multizellulärer) Wirtsorganismus, welche bzw. welcher das erfindungsgemäße Expressionssystem (d. h. mindestens einen Expressionsvektor) enthält. Die Erfindung umfasst dabei transiente Transfektanten (z. B. durch mRNA-Injektion), Wirte (Wirtszellen oder -organismen), in denen das Expressionssystem als Plasmid oder künstliches Chromosom enthalten ist, sowie Wirte in denen das Expressionssystem stabil in das Genom des Wirtes (oder einzelner Zellen des Wirtes) integriert ist. Die Wirtszelle ist bevorzugt ausgewählt aus Prokaryonten oder Eukaryonten. Bevorzugte Prokaryonten sind ausgewählt aus z. B. *Escherichia coli*- oder *Bacillus satbtilis*-Stämmen. Bevorzugte Eukaryonten sind Hefezellen (z. B. *Saccharomyces cerevisiae*, *Pichia pastoris*), Insektenzellen, amphibische Zellen oder Säugetierzellen, wie z. B. CHO, HeLa, HEK293. Bevorzugte Wirtsorganismen sind Pflanzen, wie z. B. Mais oder Tabak, Invertebraten oder Vertebraten, insbesondere *Bovidae, Drosophila melanogaster*, *Caenorhabditis elegans, Xenopus laevis,* Medaka, Zebrafisch oder *Mus musculus*, oder Zellen oder Embryonen der genannten Organismen.

Gegenstand der Erfindung ist daher auch eine Wirtszelle, die eine erfindungsgemäße Gensequenz (kodierend für ein erfindungsgemäßes Fusionsprotein) und/oder einen erfindungsgemäßen Expressionsvektor enthält.

Über einen geeigneten Promotor wird die Expression des erfindungsgemäßen Fusionsproteins in der Wirtszelle bewirkt. Zu diesem Zweck steht der Promotor unter der Kontrolle eines regulatorischen Gens, das entweder im Genom der Wirtszelle oder auf dem Expressionsvektor vorhanden ist. Dabei erfolgt die Expression konstitutiv oder bevorzugt induzierbar. Die Expression des Fusionsproteins wird bevorzugt dann gezielt induziert, wenn eine bestimmte Zahl an Wirtszellen erreicht ist. Diese Induktion erfolgt beispielsweise im Fall der *lac-, lpp-*und darauf basierenden Hybridpromotoren durch die Gabe von IPTG, im Fall des *trp*-Promotors durch Tryptophan-Mangel oder die Gabe von Indolacrylsäure, im Fall des *araBAD*-Promotors durch die Gabe von Arabinose, im Fall des *tetA*-Promotors durch die Gabe von Tetracyclin oder im Fall des *nar*-Promotors durch die Gabe von Nitrat-Ionen. Alternativ wird die Expression auch thermisch, d.h. durch Temperaturveränderung, induziert, indem die verwendeten Promotoren unter die Kontrolle von temperatursensitiven Repressoren gestellt werden, die bei Temperaturänderung die Promotoraktivität nicht mehr reprimieren können.

Für die Expression des Fusionsproteins in einer prokaryotischen Wirtszelle enthält der Expressionsvektor bevorzugt außerdem 6 bis 7 Nukleotide stromaufwärts von dem das Fusionsprotein kodierenden Nukleinsäureabschnitt eine Shine-Dalgarno-Sequenz, die zusammen mit der kodierenden Sequenz transkribiert wird und in vielen prokaryotischen Wirtszellen den Startpunkt für die Translation durch das Ribosom bildet. Bevorzugt enthält der Expressionsvektor außerdem stromabwärts von dem das Fusionsprotein kodierenden Nukleinsäureabschnitt einen Transkriptionsterminator, der gleichzeitig den Endpunkt der Transkription darstellt und in der transkribierten mRNA Sekundärstrukturen ausbildet, die die Degradation der mRNA verzögern und ihre Halbwertszeit verlängern. Weitere bevorzugte Bestandteile des Expressionsvektors sind ein Gen, das die Resistenz der Wirtszelle gegen ein Antibiotikum vermittelt und so die Selektion von Wirtszellen ermöglicht, die den Expressionsvektor aufgenommen haben, und eine ori-Sequenz (*origin of replication*; Replikationsursprung), durch welche die Replikation des Expressionsvektors durch die Replikationsmaschinerie der Wirtszelle ermöglicht wird.

Die Erfindung umfasst auch ein Verfahren zur Herstellung eines Zielproteins (d. h. eines zu exprimierenden Proteins), das die folgenden Verfahrensschritte umfasst:

a) Exprimieren eines erfindungsgemäßen Fusionsproteins, welches das Zielprotein als Fusionspartner umfasst, in einer Wirtszelle, und

b) Inkontaktbringen des Fusionsproteins mit einer $Ca^{2+}$-Lösung und Induzierung der Spaltung des autokatalytischen Proteinlinkers, wodurch das Fusionsprotein gespalten und das Zielprotein freigesetzt wird.

Die Aufreinigung des Fusionsproteins aus der Wirtszelle erfolgt ebenfalls über gängige Methoden der Molekularbiologie und der Proteinbiochemie. Dabei sind die Methoden abhängig von der Art der verwendeten Fusionspartner. Entscheidend dabei ist, dass während der Aufreinigung bis zu dem Zeitpunkt, wo das Fusionsprotein wunschgemäß gespalten werden soll, keine freien $Ca^{2+}$-Ionen im Medium vorhanden sind. Dies wird beispielsweise durch den Einsatz von Chelatbildnern sichergestellt, die vorhandene zweiwertige Ionen binden. Bevorzugte Chelatbildner sind Ethylendiamintetraessigsäure (EDTA), Ethylenglycoltetraessigsäure (EGTA), Nitrilotriacetat (NTA), Diethylentriaminpentaessigsäure (DTPA), oder Phosphonate wie beispielsweise 1-Hydroxyethan-(1,1-diphosphonsäure) (HEDP), Aminotrimethylenphosphonsäure (ATMP) bzw. Nitrolo-tris(methylenphosphonsäure) (NTMP), Diethylentriaminpenta(methylen-phosphonsäure) (DTPMP), Ethylendiamintetra(methylenphosphonsäure) (EDTMP) und 2-Phosphonobutan-1,2,4-tricarbonsäure (PBTC).

Zur Induktion der Spaltung ist vorteilhaft nur die Zugabe von $Ca^{2+}$- Ionen notwendig. Das molare Verhältnis zwischen der Menge an eingesetzten $Ca^{2+}$-Ionen zu der eingesetzten Proteinmenge beträgt dabei bevorzugt zwischen 0,1 fach und 10000 fach, vorzugsweise 1fach bis 1000fach, besonders bevorzugt 10fach bis 100fach (Ionen : Protein).

Dabei beträgt die eingesetzte freie $Ca^{2+}$-Ionenkonzentration im Spaltungsansatz bevorzugt zwischen 100 nmol/l und 500 mmol/l, besonders bevorzugt 0,01 bis 50 mmol/l. Vorteilhafterweise kann die autoproteolytische Spaltung auch in Gegenwart von Chelatbildnern, die vorhandene zweiwertige Ionen binden (wie z. B. EDTA), durchgeführt werden, solange sichergestellt ist, dass in der Lösung ausreichend freie $Ca^{2+}$-Ionen vorhanden sind. Dies kann beispielsweise durch einen entsprechenden Überschuss an $Ca^{2+}$-Ionen erreicht werden. Da die Aufreinigung des Fusionsproteins in der Regel unter Zusatz von Chelatbildnern durchgeführt werden wird, macht dies vorteilhaft die Durchführung eines zeitaufwändigen Dialyseschritts zur Entfernung des Chelatbildners vor der Induzierung der Spaltung überflüssig.

Der autokatalytische Proteinlinker schneidet vorteilhaft nur *in cis* d.h. er spaltet nur sich selbst. Andere Sequenzen in fremden Peptiden oder Proteinen, welche die Sequenz der Spaltstelle DP oder EP umfassen, spaltet er nicht, eine *in trans* Proteolyse ist also nicht möglich. Dadurch werden vorteilhafterweise andere während der Spaltung anwesende Proteine durch den autokatalytischen Proteinlinker nicht angegriffen. Ein weiterer Vorteil des autokatalytischen Proteinlinkers ist außerdem, dass auch innerhalb der eigenen Sequenz eine potentielle Spaltstelle nur erkannt wird, wenn sie sich in der richtigen Anordnung zum katalytischen Zentrum des Proteinlinkers befinden. Dadurch ist der Proteinlinker für die Verknüpfung mit jeder gewünschten Proteinsequenz geeignet, ohne dass der Fusionspartner durch die proteolytische Aktivität angegriffen wird.

Der autoproteolytische Proteinlinker ist außerdem vorteilhaft sehr hitzestabil und spaltet sich selbst noch nach 10-minütiger Inkubation bei 50 °C nach Induktion durch $Ca^{2+}$-Ionen vollständig. Nach 10-minütiger Inkubation bei 70 °C findet zumindest noch eine teilweise Spaltung statt. Diese vorteilhafte Eigenschaft kann in Kombination mit hitzestabilen Proteinen genutzt werden, z. B bei der Aufreinigung von hitzestabilen Enzymen.

Die Erfindung umfasst desweiteren ein Verfahren zur Herstellung und Aufreinigung eines Zielproteins, welches die folgenden Verfahrensschritte umfasst:

a) Exprimieren des erfindungsgemäßen Fusionsproteins, welches einen Affinitätstag und das Zielprotein als Fusionspartner umfasst, in einer Wirtszelle, und

b) Inkontaktbringen des Fusionsproteins mit einem Molekül, das spezifisch an den Affinitäts-Tag bindet, wobei das Molekül an einer Matrix immobilisiert ist,

c) Inkontaktbringen des rekombinanten Fusionsproteins mit einer $Ca^{2+}$-Lösung und Induzierung der Spaltung des autokatalytischen Proteinlinkers, wobei das Zielprotein freigesetzt wird.

Das Zielprotein kann anschließend durch Spülen der Matrix mit einer geeigneten Pufferlösung erhalten werden.

Die Matrix, die für die Affinitätschromatographie als stationäre Phase dient und an der das den Affinitäts-Tag spezifisch bindende Molekül immobilisiert ist, ist bevorzugt eine für die Affinitätschromatographie geeignete Matrix, wie zum Beispiel Dextran, Cellulose, Agarose bzw. Agarosederivate wie z. B. quervernetzte Agarose, Polyacrylamid, Methacrylat, Polystyrol, Kieselsäure, Hydroxylapatit, magnetische Partikel oder poröse Glaskügelchen.

Vorteilhaft wird im erfindungsgemäßen Verfahren keine Agglomeratbildung oder Bildung von Vernetzungsprodukten beobachtet, d.h. die bei der Spaltung der Zielsequenz DP freiwerdende C-terminale Aminosäure Aspartat wird nicht kovalent an einen Lysinrest eines anderen Proteins gebunden. Der Einsatz von Thiolverbindungen und/oder Reduktionsmitteln ist im erfindungsgemäßen Verfahren daher weder zur Induktion der Proteinspaltung noch zur Vermeidung von Agglomeraten bzw. Vernetzungsprodukten notwendig.

Das Verfahren wird vorzugsweise in einem pH-Bereich von pH 4 bis pH 10, bevorzugt pH 5 bis pH 9, besonders bevorzugt pH 6 bis pH 8 durchgeführt. Ein zusätzlicher Vorteil des erfindungsgemäßen Verfahrens ist die hohe Geschwindigkeit der Proteolyse, wodurch zeitsparend sehr kurze Proteolyseschritte ermöglicht werden. Der Proteolyseschritt, d.h. die Inkubation des erfindungsgemäßen Fusionsproteins mit einer $Ca^{2+}$-Lösung, wird bevorzugt zwischen 1 Minute und 2 h, besonders bevorzugt zwischen 5 Minuten und 1 h ausgeführt. Diese kurzen Inkubationszeiten, von bevorzugt unter 10 Minuten, besonders bevorzugt unter 5 Minuten, führen vorteilhaft bereits bei Raumtemperatur zu einer vollständigen Spaltung. Eine Verlängerung des Proteolyseschritts führt jedoch zu keinen unerwünschten Nebenprodukten, sodass dieser Schritt auch länger als 2 h durchgeführt wird kann, ohne zu unspezifischen Spaltprodukten zu führen. Vorteilhaft ist die Proteolyse auch bei Temperaturen unter 10 °C, bevorzugt bei 0 °C bis 4 °C möglich und wird dadurch auch nicht deutlich langsamer. Dies ermöglicht die Durchführung der Proteolyse auch unter Kühlung, was bei solchen Fusionspartnern, die bereits bei Raumtemperatur leicht degradiert werden, besonders vorteilhaft ist.

Ein weiterer Vorteil des erfindungsgemäß verwendeten Proteinlinkers besteht darin, dass seine autokatalytische Aktivität durch viele gängige Proteaseinhibitoren nicht beeinträchtigt wird. Dies ermöglicht vorteilhaft den Einsatz von Proteaseinhibitoren während des erfindungsgemäßen Verfahrens und bei der Lagerung des erfindungsgemäßen Fusionsproteins, wodurch ein unerwünschter proteolytischer Abbau des Fusionsproteins durch Proteaseverunreinigungen, die beispielsweise aus der Wirtszelle stammen, gehemmt und/oder verhindert wird. Proteaseinhibitoren, welche die autokatalytische Spaltung des erfindungsgemäßen Fusionsproteins nicht stören, sind bevorzugt ausgewählt aus Serinprotease-Inhibitoren, wie z.B. Aprotinin, 4-(2-Aminoethyl)-benzolsulfofluorid (AEBSF), ε-Amino-n-capronsäure oder Phenylmethylsulfonylfluorid (PMSF), Cysteinprotease-Inhibitoren, wie z.B. Leupeptin oder Antipain, und Metalloprotease-Inhibitoren, z.B. 1,10-Phenanthrolin.

Bestandteil der Erfindung ist auch ein Kit bestehend aus einem Expressionsvektor und einer $Ca^{2+}$-Lösung, der zur Herstellung eines erfindungsgemäßen Fusionsproteins und zur Durchführung der autoproteolytischen Katalyse verwendet wird. Dieser Kit enthält bevorzugt als weitere Bestandteile:

- Substanzen für die Klonierung der für den oder die Fusionspartner kodierenden Nukleinsäuresequenzen in den Expressionsvektor, wie z. B. Restriktionsenzyme, Ligase, Puffer,

- Substanzen oder auch Vorrichtungen für das Einbringen des Expressionssystems in die Wirtszelle, wie z. B. Puffer, Shotgun- oder Elektroporationsgeräte,

- eine Wirtszelle,

- Substanzen oder auch Vorrichtungen für den Aufschluss der Wirtszelle und für die Aufreinigung des Fusionsproteins, wie z.B. Puffer, Protease-Inhibitor-Lösungen, Filter, Ultraschallgeräte, Affinitätsmatrizes.

Substanzen, die mit dem Fusionsprotein nach dem Aufschluss der Wirtszelle mit dem Fusionsprotein in Kontakt kommen, sind bevorzugt frei von $Ca^{2+}$. Besonders bevorzugt umfassen diese Substanzen einen Chelatbildner wie beispielsweise EDTA, EGTA, NTA, DTPA, oder Phosphonate wie beispielsweise HEDP, ATMP, NTMP, DTPMP, EDTMP und PBTC.

Die Erfindung wird erläutert durch die folgenden Figuren und Ausführungsbeispiele, ohne auf diese beschränkt zu sein.

**Figuren:**

**Figur 1** zeigt ein Alignment der Sequenzen SEQ ID No. 1 bis 8 einschließlich der Konsensussequenz dieser Sequenzen. Konservierte Motive sind hervorgehoben.

Die folgenden Symbole (Consensus Symbole) beschreiben den Grad der Konservierung in der jeweiligen Spalte:

"*" - Die Aminosäure in dieser Spalte ist identisch in allen Sequenzen des Alignments.

":" - In dieser Spalte wurden konservierte Substitutionen beobachtet oder die Aminosäure fehlt in einer Sequenz.

"." - In dieser Spalte wurden teilweise konservierte Substitutionen beobachtet.

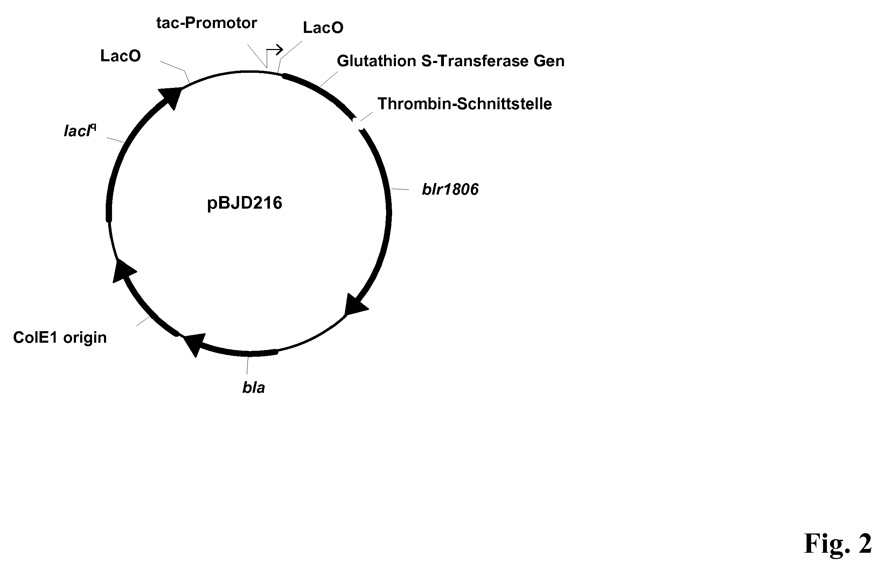

**Figur 2** zeigt eine schematische Darstellung des Expressionsvektors pBJD216. Das durch Insertion des Gens *blr1806* in den Expressionsvektor pGEX4-T3 entstandene Plasmid pBJD216 kodiert für ein Fusionsprotein aus GST (N-terminal) und Blr1806 (C-terminal), dessen Expression unter der Kontrolle des *tac*-Promotors steht. Desweiteren enthält das Plasmid einen *E. coli*-Replikationsursprung (ColE1 origin) und ein Gen *(bla)*, welches Ampicillin-Resistenz vermittelt.

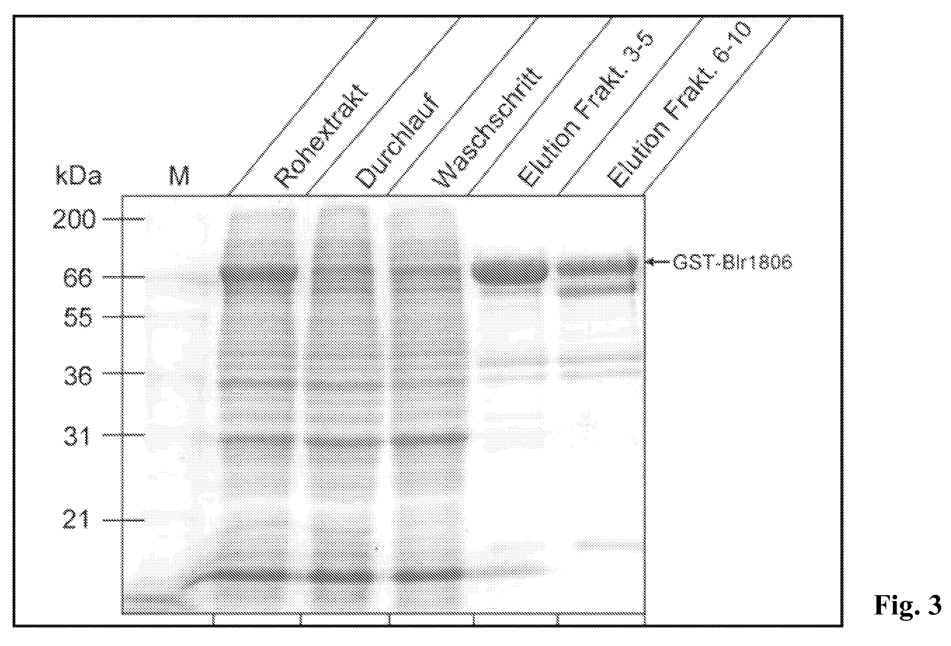

**Figur 3** zeigt die Reinigung des in *E. coli* exprimierten Fusionproteins GST-Blr1806 durch Säulenchromatographie in einer SDS-PAGE der gesammelten Fraktionen. Im "Rohextrakt" ist das überexprimierte Fusionsprotein als deutliche Bande zu sehen, die dem erwarteten Molekulargewicht von 77 kDa entspricht. Nach der Elution wurden die Elutionsfraktionen 3 bis 5 sowie 6 bis 10 vereinigt.

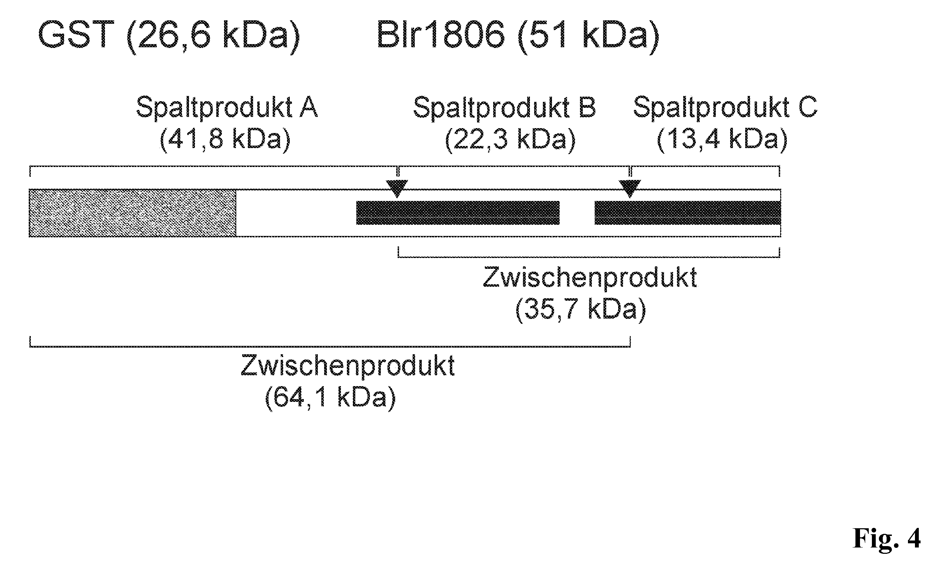

**Figur 4** zeigt eine schematische Darstellung von GST-Blr1806. Der GST-Anteil ist als schraffierte Box, der Blr1806-Teil als weiße Box mit den DUF 1521-Domänen (schwarze Boxen) und den Spaltstellen (schwarze Dreiecke) dargestellt. Angegeben sind außerdem die Fragmentgrößen der Spaltprodukte und Zwischenprodukte.

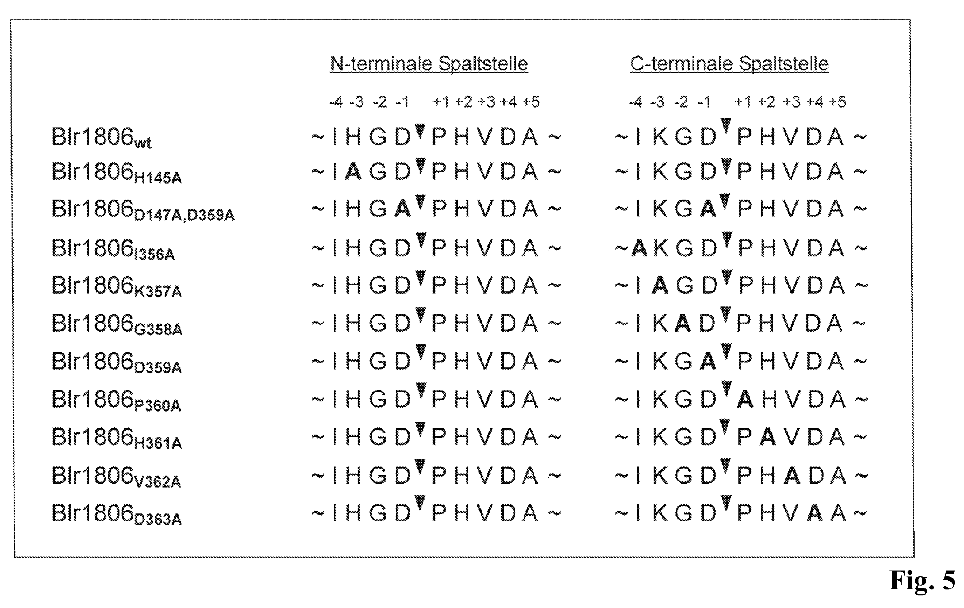

**Figur 5** zeigt einen Überblick über die gerichteten Aminosäureaustausche in Blr1806. Dargestellt sind jeweils die vier N- und fünf C-terminal neben den Spaltstellen gelegenen Aminosäuren, gekennzeichnet in Relation zur Spaltstelle. Blr1806wt zeigt die Originalsequenz. Die tiefgestellten Ziffern in der Beschriftung Blr1806 bezeichnen die abweichenden Aminosäurepositionen in Blr1806. Die veränderten Aminosäuren sind fett gedruckt. Die Spaltstellen sind durch schwarze Dreiecke gekennzeichnet.

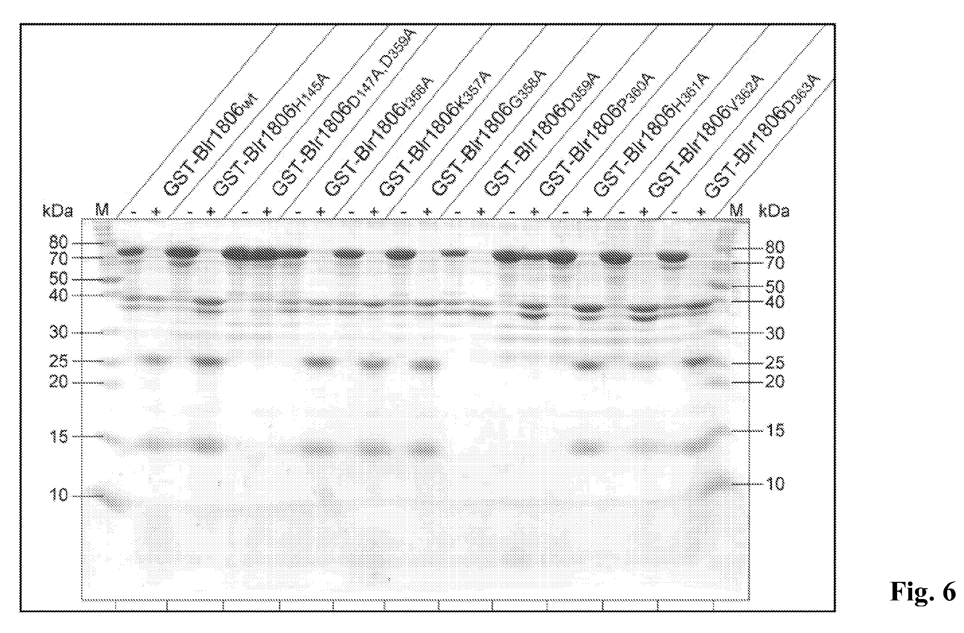

**Figur 6** zeigt eine SDS-PAGE zur Analyse der autoproteolytischen Aktivität der Derivate von GST-Blr1806 mit gerichteten Aminosäureaustauschen. Es wurde jeweils das Volllängenprotein ohne $CaCl_2$-Behandlung (-) und das mit $CaCl_2$-Lösung behandelte Protein (+) nebeneinander aufgetragen.

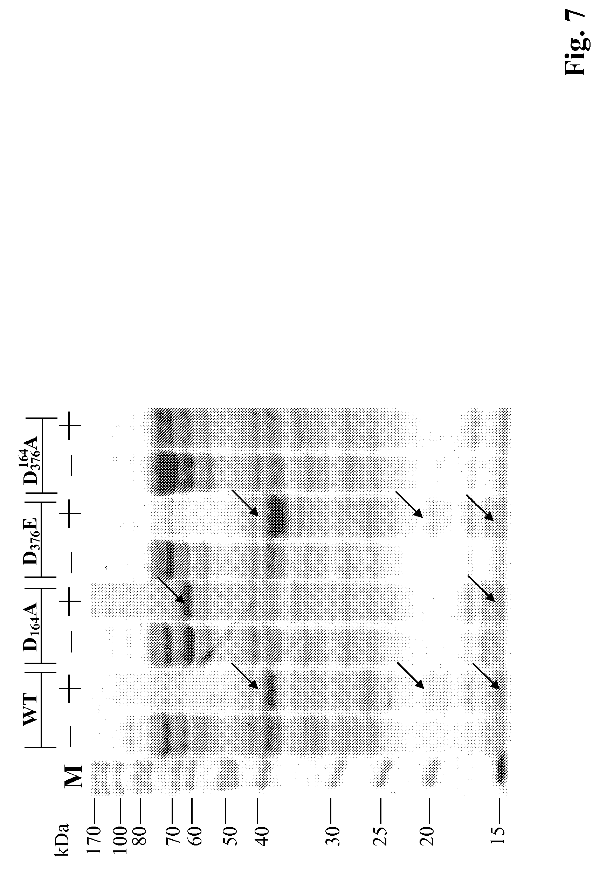

**Figur 7** zeigt eine SDS-PAGE von GST-Blr1649 und GST-Blr1649-Derivaten. Es wurden je 25 µg Protein ohne $CaCl_2$-Behandlung (-) und mit $CaCl_2$-Behandlung (+) nebeneinander auf das Gel aufgetragen. Die Pfeile markieren Spaltprodukte.

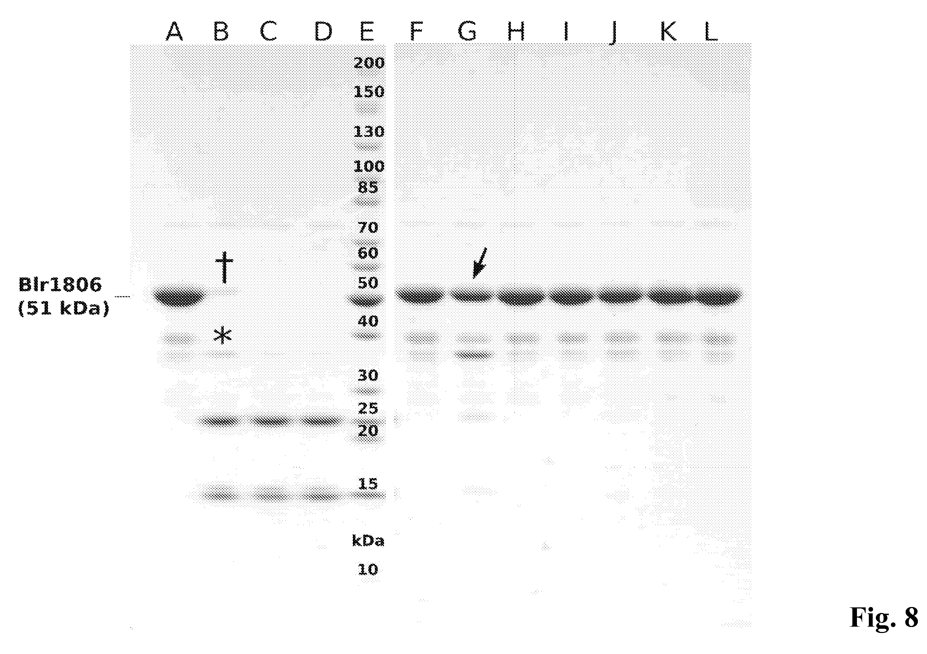

**Figur 8** zeigt eine SDS-PAGE von Blr1806 nach Inkubation mit verschiedenen Metallionen. Die Proteine wurden mit gepufferten Metallsalz-Lösungen (pH 6,8) 10 min bei RT inkubiert. Die Metallionen wurden danach durch Aufkochen in EDTA-haltigem Probenpuffer gebunden. Konzentration der Komponente (= Konzentration freier Metallionen): **A** Puffer (0,125 mol/l Tris, pH 6,8); **B** 0,5 mmol/l $CaCl_2$; **C** 1 mmol/l $CaCl_2$; **D** 2 mmol/l $CaCl_2$; **E** Molekular-gewichtsstandard; **F** 2 mmol/l $ZnSO_4$; **G** 2 mmol/l $ZnSO_4$; 2 mmol/l $CaCl_2$; **H** 2 mmol/l $MgCl_2$; **I** 2 mmol/l $CuCl_2$; **J** 2 mmol/l $FeSO_4$; **K** 2 mmol/l $NiCl_2$; **L** 2 mmol/l $CoCl_2$.

Bei einer Konzentration von freiem Calcium von 0,5 mmol/l ist noch eine schwache Bande des Volllängen-Proteins erkennbar (Spur B, Kreuz). Bei höheren Konzentrationen verschwindet diese Bande (Spur C und D). Die 37 kDa-Bande ist bei 0,5 mmol/l Calcium etwas stärker als bei 1 mmol/l und 2 mmol/l (Sternchen). Die anderen Metallionen führen keine Fragmentierung von Blr1806 herbei (Spuren F-L). Mit $ZnSO_4$ lässt sich die $Ca^{2+}$-bedingte Fragmen-tierung soweit aufhalten, dass die Volllängenbande fast gänzlich erhalten bleibt (Spur G, Pfeil). Es wurde ein PA-Gradientengel (4%-20%) verwendet. Von den Ansätzen (1 $\mu$g/$\mu$l Protein) wurden 20 $\mu$l aufgetragen. Bei der Zn-Ca-Doppelinkubation wurde erst $ZnSO_4$ und danach $CaCl_2$ zugegeben.

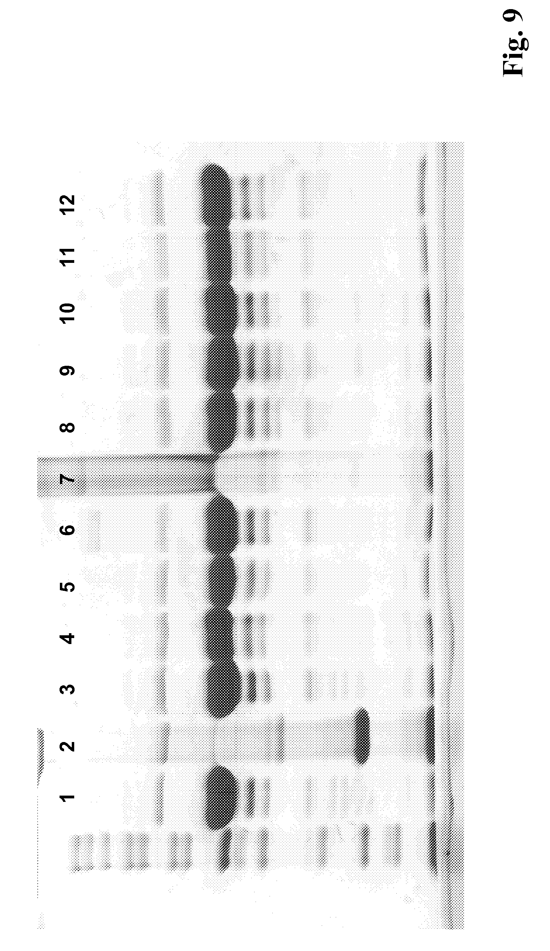

**Figur 9** zeigt eine weitere SDS-PAGE von Blr1806 nach Inkubation mit verschiedenen Metallionen. Von den Metallsalzen wurde eine Endkonzentration von 25 mM eingesetzt. 6 $\mu$g Blr1806 wurden in der gepufferten Metallsalzlösung 10 Minuten bei Raumtemperatur inkubiert. Die Metallionen wurden danach durch Aufkochen in EDTA-haltigem Probenpuffer gebunden und die Ansätze wurden auf das Gel aufgetragen. In den einzelnen Spuren finden sich die Proteine aus folgenden Ansätzen: **1** - ohne Zusatz von Metallsalzen; **2 -** $CaCl_2$; 3 - Tris (Kontrolle); **4 -** $CdCl_2$; **5 -** $CoCl_2$; **6 -** $CuCl_2$; 7 - $FeSO_4$; **8 -** $MgCl_2$; **9 -** $MnCl_2$; **10 -** $MnSO_4$; **11 -** $NiCl_2$; **12 -** $ZnSO_4$.

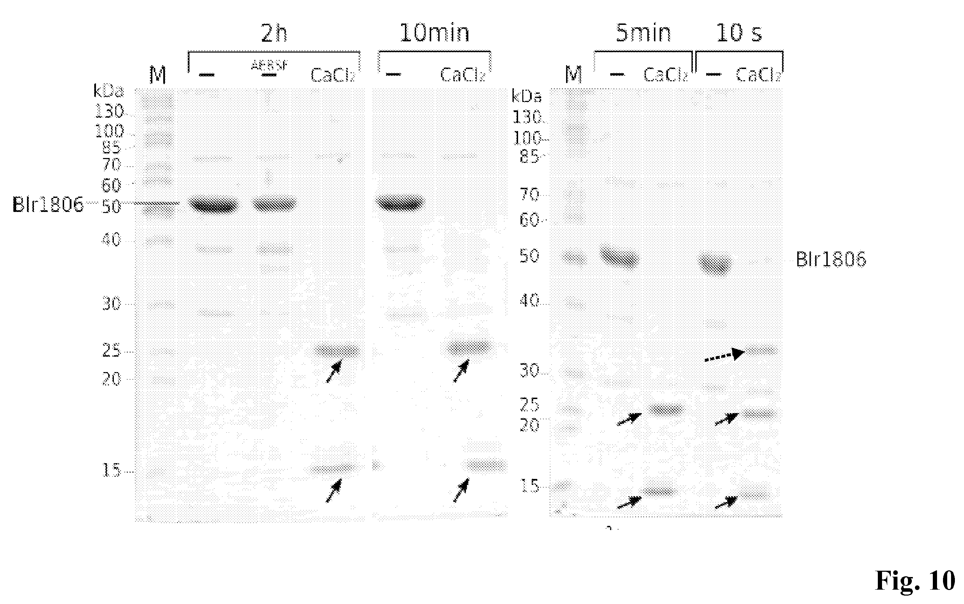

**Figur 10** zeigt eine SDS-PAGE von Blr1806 nach unterschiedlicher Dauer der $Ca^{2+}$-Inkubation. Pro Spur wurden 8 $\mu$g Protein aufgetragen. Ansätze von Blr1806 mit $CaCl_2$ (gepuffert, freie Ionen: ca. 2 mM) oder Ca-freiem Puffer (-) wurden mit verschiedener Dauer bei RT inkubiert. Banden, welche nur bei der Inkubation mit $CaCl_2$ auftreten, sind durch Pfeile hervorgehoben. Durch die längeren Inkubationszeiten entstehen keine zusätzlichen Fragmente. Bei einer sehr kurzen Inkubation (10 s) ist eine zusätzliche Bande erkennbar (gestrichelter Pfeil). Beim Zusatz von AEBSF und ohne $CaCl_2$-Behandlung ändert sich das Bandenmuster nicht.

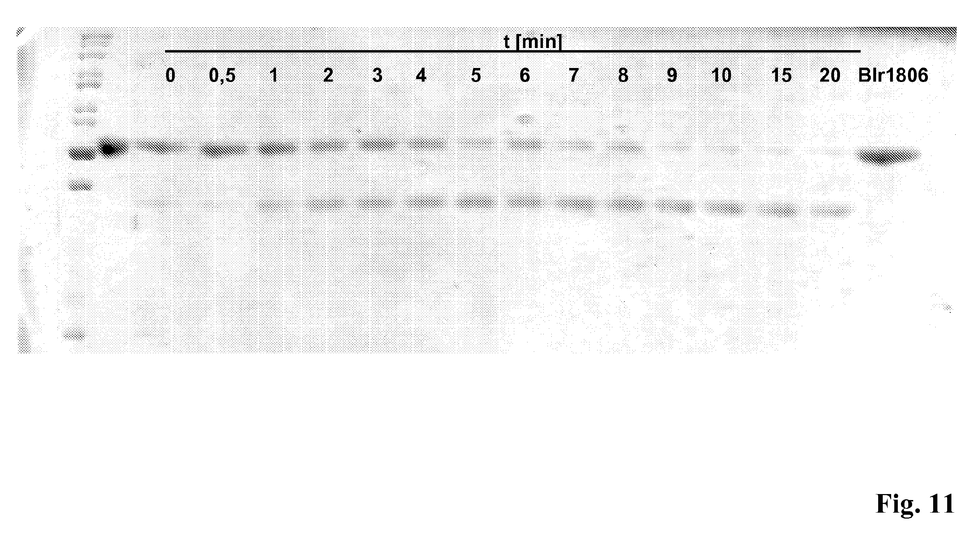

**Figur 11** zeigt eine SDS-PAGE von Blr1806 nach unterschiedlicher Dauer der $Ca^{2+}$-Inkubation bei 0 °C. Probennahme erfolgte nach 0, 0,5, 1, 2, 3, 4, 5, 6, 7, 8, 9, 10, 15 und 20 Minuten. Als Kontrolle wurde Volllängen-Br1806 aufgetragen (ganz rechts).

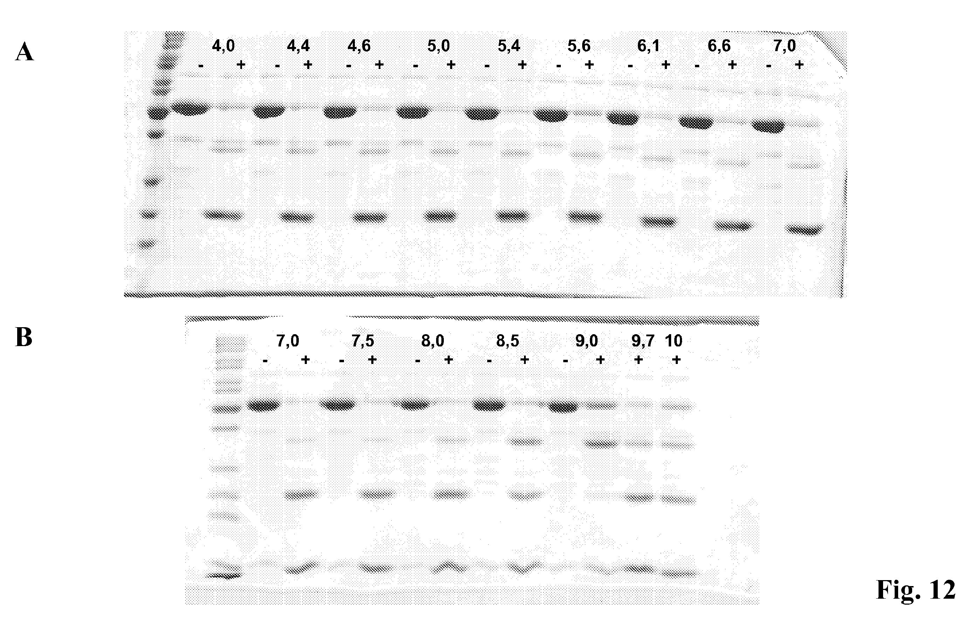

**Figur 12** zeigt eine SDS-PAGE nach $Ca^{2+}$-Inkubation von Blr1806 bei verschiedenen pH-Werten. Eine Präparation von Blr1806 (durchgeführt mit PBSE-Puffer) wurde mit Pufferlösungen mit unterschiedlichen pH-Werten inkubiert (40 min bei Raumtemperatur). Die Lösungen wurden mit (+) oder ohne (-) $CaCl_2$ angesetzt.

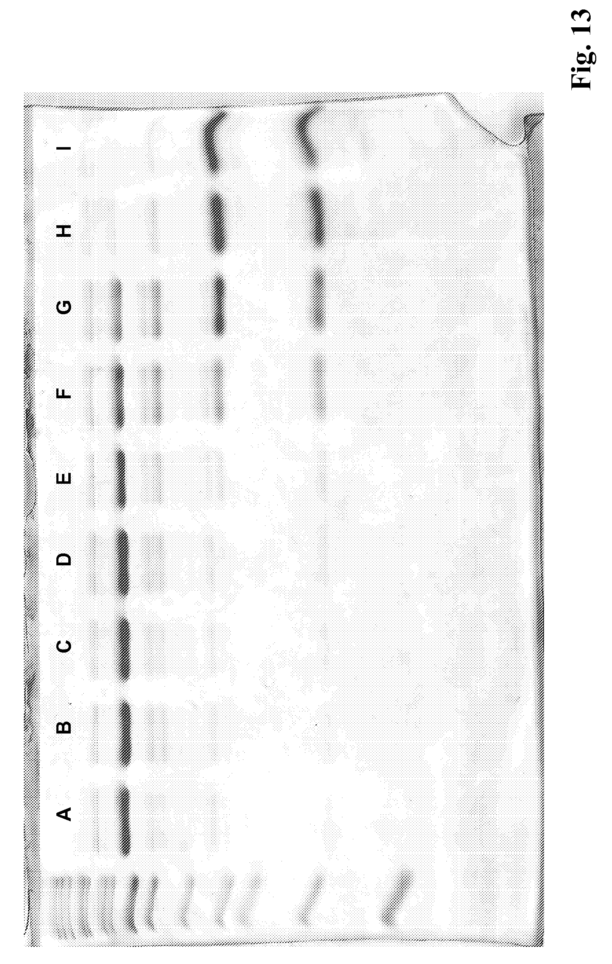

**Figur 13** zeigt eine SDS-Page nach $Ca^{2+}$-Inkubation von Blr1806 in Anwesenheit von 10 mM EDTA mit unterschied-lichen $Ca^{2+}$-Konzentrationen (**A**: 0,1 mM, **B**: 0,2 mM, **C**: 0,4 mM:, **D**: 0,7 mM, **E**: 1,4 mM, **F**: 2,8 mM, **G:** 5,7 mM, **H:** 11,4 mM, **I:** 22,7 mM).

**Ausführungsbeispiele:**

**Ausführungsbeispiel 1 a) Expression und Reinigung des GST-Blr1806-Fusionsproteins**

Zur Aufreinigung des GST-Blr1806-Fusionsproteins wird der in Figur 2 gezeigte Expressionsvektor pBJD216 verwendet, der die kodierenden Sequenzen von *blr1806* und dem Gen der Glutathion-S-Transferase (*gst*) von *Schistosoma japonicum* in einem durchgehenden offenen Leserahmen enthält.

```
MSPILGYWKIKGLVQPTRLLLEYLEEKYEEHLYERDEGDKWRNKKFELGLEFPNL
PYYIDGDVKLTQSMAIIRYIADKHNMLGGCPKERAEISMLEGAVLDIRYGVSRIA
YSKDFETLKVDFLSKLPEMLKMFEDRLCHKTYLNGDHVTHPDFMLYDALDVVLYM
DPMCLDAFPKLVCFKKRIEAIPQIDKYLKSSKYIAWPLQGWQATFGGGDHPPKSD
LVPRGSPNSMQYLPVAGLPVVGAPDSMNGVAPEGAVVTPTFNAMLGQYAPASYQY
LPVASPALVGTAVGSVVPVVVAPAVVTTQAYMMAPPPSTMLMSQLNQNAEPPVDP
VWTHEVKDGKATINLGDKYTITANEKDGTWTVRNNQTGHVTVIHGDPHVDADGDG
KDDFDFKKDMTFQLDDGTKITVNNVDYGNGQAISSKLTITNGHNAIVVEGLGDDK
DGKNNLRVTQSNAGMTLDELTSDGAQTIHESGQGWVDGAGRAVNQASIDDGEQGR
GPGNYQYASGAPTSTAPVFFVPPPPPPLAMVPVAAAQVPTQSSQPAPVWSHEVRD
GKAEIKLGDKYSILVDENDGTVLIRNSQTGKITSIKGDPHVDADGDGKVDFDFKK
NMTFQLDDGTKITVDTVDIGKGKTMASKLTITNGDNAMVVEGLGDRFDGKNNLKV
TQSNAGRTLDQLTSDGAQTIYEQPGSGWVDRSGRQVNQEIIDSNENPGTTSDA*
```

**Aminosäuresequenz des GST-Blr1806-Fusionsproteins (SEQ ID No. 14)**

Hierbei steht die *gst-blr1806*-Fusion unter der Kontrolle des starken *tac*-Promotors. Der Vektor kodiert außerdem für den LacI-Repressor und enthält LacO-Operatorstellen, sodass die Expression des *gst-blr1806*-Konstrukts durch Zugabe von IPTG zur Kultur induziert werden kann. Zwischen den Gensequenzen des Fusionskonstruktes befindet sich eine Sequenz, welche eine Spaltstelle für die Proteinase Thrombin kodiert. Der *E. coli*-Expressionsstamm BL21(DE3) wird durch Elektroporation mit dem Expressionsvektor pBJD216 transformiert.

Die Zellen aus 400 ml Kulturvolumen des *E. coli*-Expressionsstammes BL21(DE3) pBJD216 werden in 25 ml Aufschlusspuffer (50 mmol/l Tris-Cl (pH 8,0 bei 6°C); 200 mmol/l KCl; 10 mmol/l EDTA) aufgeschlossen. Dazu wird der Suspension 10fach-Lysozymstammlösung zu einer einfachen Endkonzentration zugegeben. Nach Inkubation auf Eis (30 min) wird der Zellaufschluss durch den Einsatz der Ultraschallsonde fortgesetzt. Dabei wird die im Eis gelagerte Suspension dreimal für 10 s bei einer Schallintervalleinstellung von 6/10 und einer Leistung von 75% behandelt. Anschließend wird die Suspension 4 min zum Abkühlen auf Eis belassen. Die Behandlung wird einmal wiederholt. Unlösliche Bestandteile werden durch Zentrifugation pelletiert. 5 ml dieses Proteinrohextraktes, d.h. ca. 11 mg Gesamtprotein, werden zur Reinigung des GST-Blr1806-Fusionsproteins mittels Affinitätschromatographie an einer Sepharose-Säule mit immobilisiertem Glutathion eingesetzt. Das mittels Elutionspuffer (100 mmol/l Tris-Cl (pH 8,0 bei 6°C), 10 mmol/l EDTA, 10 mmol/l reduziertes Glutathion) von der Säule eluierte GST-Blr1806-Fusionsprotein wird in Fraktionen von 250 μl bzw. 500 μl aufgefangen. Nach Analyse der Elutionsfraktionen mittels SDS-PAGE werden die Elutionsfraktionen sinnvoll vereinigt (Figur 3, Fraktionen 3-5) und weiterverwendet. Insgesamt werden 0,25 mg Protein mit der gewünschten Reinheit von der Säule eluiert. Während in den Elutionsfraktionen 3-5 das Fusionsprotein größtenteils als Volllängenprotein vorliegt, enthalten die Elutionsfraktionen 6-10 einen relativ hohen Anteil bereits hydrolysierten Proteins (Figur 3). Es sind Banden zu sehen, die den Molekulargewichten der Spaltprodukte (14 und 41 kDa) bzw. der Zwischenprodukte (64 und 36 kDa) zugeordnet werden können.

**Ausführungsbeispiel 1 b) Bestimmung der Spaltstellen von Blr1806**

Das *Bradyrhizobium japonicum*-Protein Blr1806 enthält zwei DUF 1521-Domänen, die sich im Bereich der Aminosäurereste 103 bis 272 bzw. 315 bis 484 befinden. Die Bestimmung der genauen Spaltstellen von Blr1806 erfolgte durch N-terminale Proteinsequenzierung der Spaltprodukte B und C (Figur 4) des heterolog in *E. coli* exprimierten und durch Säulenchromatographie gereinigten Fusionsproteins GST-Blr1806.

Die Proteolyse von 40 μg Protein in 150 μl Puffer (50 mmol/l Tris, 10 mmol/l EDTA, pH 7,7) erfolgte durch Zugabe von 5 μl 0,5 mol/l CaCl$_2$ in 125 mmol/l Tris und 10-minütiger Inkubation bei RT. Zur Konzentrierung wurde das Protein mit Aceton präzipitiert. Durch SDS-PAGE wurden die Spaltprodukte aufgetrennt und mittels des *semi dry blotting*-Verfahrens mit einem Glycin-freien, diskontinuierlichen Tris-CAPS-Puffersystem auf eine PVDF-Membran übertragen. Die Analyse erfolgte durch die Firma Chroma Tec (Greifswald). Die Sequenzierung der jeweils 5 N-terminalen Aminosäuren ergab für beide Spaltprodukte die Aminosäuresequenz Pro-His-Val-Asp-Ala. Daraus ergeben sich die Spaltstellen nach Position 147 und 359 der Aminosäuresequenz von Blr1806 (also jeweils nach dem Aminosäurerest D45 der DUF 1521-Domäne) und die berechneten Fragmentgrößen der Spaltprodukte von 41,8 kDa (Fragment A einschließlich GST), 22,3 kDa (Fragment B) und 13,4 kDa (Fragment C) (siehe Figur 4).

**Ausführungsbeispiel 2 a) Modifikation der Spaltstellensequenz von Blr1806 und Analyse der Autoproteaseaktivität**

Um zu klären, welche Aminosäuren für die Erkennung der Spaltstellen von Blr1806 erforderlich sind, wurde ein systematischer Aminosäureaustausch im Bereich der Spaltstellen durchgeführt.

Durch *in vitro*-Punktmutagenese wurden 9 Derivate von *blr1806* hergestellt, in deren Genprodukten je eine Aminosäure in der Umgebung der Spaltstelle durch Alanin ersetzt wurde (Figur 5). Als Ausgangsmolekül für die Punktmutagenese-Reaktion wurde das Plasmid pBJD216 verwendet, welches für ein GST-Blr1806-Fusionsprotein kodiert (Figur 2). Oligodesoxynukleotide, die die entsprechende(n) Modifikation(en) tragen, kamen als Primer zum Einsatz und in einer Polymerasekettenreaktion wurde das entsprechende pBJD216-Derivat amplifiziert. Anschließend erfolgte die Transformation der amplifizierten Konstrukte in den *E. coli*-Stamm DH10B. Die Punktmutationen sowie die Korrektheit der restlichen Sequenz von *blr1806* wurden durch Sequenzierung bestätigt.

Die Derivate von GST-Blr1806 wurden im *E. coli*-Stamm BL21(DE3) exprimiert und durch das "*Batch*"-Verfahren unter Verwendung von Glutathion-Sepharose gereinigt. Zellkultur, Aufschluss und Reinigung erfolgten nach Standardmethoden.

Die Analyse der Fragmentgrößen nach erfolgter Proteolyse durch Inkubation mit $CaCl_2$ erfolgte mittels SDS-PAGE (Figur 6). Das Wildtyp-Protein (GST-Blr1806wt) zeigte das typische Bandenmuster. Die Fragmentgrößen sind der Figur 4 zu entnehmen. GST-Blr1806D359A und GST-Blr1806P360A zeigten gegenüber dem Wildtypprotein ein verändertes Bandenmuster. In den mit $CaCl_2$ behandelten Ansätzen fehlen die 23 kDa- und 14 kDa-Banden. Die C-terminale Spaltstelle ist in diesen Derivaten inaktiviert. Der Austausch der Aminosäure direkt vor (Position -1 in Bezug auf die Spaltstelle) bzw. direkt nach der Spaltstelle (Position +1 in Bezug auf die Spaltstelle) inaktiviert diese gänzlich. Daraufhin wurde ein Blr1806-Derivat erzeugt, in dem das Aspartat vor beiden Spaltstellen gegen Alanin ausgetauscht wurde (GST-Blr1806D147A, D359A). Dieses Protein zeigte keine autoproteolytische Aktivität und lag auch nach Inkubation mit $CaCl_2$ noch als Volllängenprotein vor. Alle anderen in dieser Arbeit erzeugten Derivate von GST-Blr1806 zeigten kein verändertes Spaltungsverhalten im Vergleich zum Wildtypprotein. Die Aminosäuren an den Positionen -4, -3, -2, sowie +2, +3 und +4 in Bezug auf die Spaltstelle haben keine Bedeutung für die Erkennung und Spaltung von Blr1806.

**Ausführungsbeispiel 2 b) Austausch der Aminosäure D gegen E in der Spaltstelle von GST-Blr1649**

Das Fusionsprotein GST-Blr1649 wurde analog zu Ausführungsbeispiel 1 hergestellt. Die Aminosäureaustausche in den GST-Blr1649-Derivaten und die Analyse der autoproteolytischen Aktivität wurden wie in Ausführungsbeispiel 2 a) beschrieben durchgeführt.

```
MSPILGYWKIKGLVQPTRLLLEYLEEKYEEHLYERDEGDKWRNKKFELGLEFPNL
PYYIDGDVKLTQSMAIIRYIADKHNMLGGCPKERAEISMLEGAVLDIRYGVSRIA
YSKDFETLKVDFLSKLPEMLKMFEDRLCHKTYLNGDHVTHPDFMLYDALDVVLYM
DPMCLDAFPKLVCFKKRIEAIPQIDKYLKSSKYIAWPLQGWQATFGGGDHPPKSD
LVPRGSPNSAYLPVAGLPVVGALDSTVNGVGSEGVVASPAFSTLLGQYVTPGAYQ
YLPAGAAVVVAPVVVPAGGVTTAVVAPAVVTPVVAAPVYMMAPPPPFMLLSQQSS
GSEPSVDPVWTHEVKDGKATINLGDKYTITANEKDGTWTVRNNQTGHVTKIHGDP
HVDANGDGKDDFDFKKDMTFQLDDGTKITVNNVDYGNGETISSRLTITNGHNAMV
VEGLGDDKDGKNNLRVTQSNAGMTLDELTPDGAQTIHESGQGWVDGAGGVVNQAS
IDAGEQGRAPGNYQSASATPASATPVFFVPPPPPPLAMVPFAVAQTPAPSSQPAP
VWSHEVKDGKATINLGDKYTITADEKDGTWTVRNNQTGHVSRIHGDPHVDANGDG
KDDFDFKKGMTFQLDDGTKITVDTVNYGKGKTISSKLTITNGDNAMVVEGLGDDK
DGKNNLKVTQSNAGRTLDQLTSDGAQTIHETNQGWVDNSGRKVTQSVINHNENPD
APPDFKTMMREAMFRRIFGRDAQPA*
```

**Aminosäuresequenz des GST-Blr1649-Fusionsproteins (SEQ ID No. 15)**

Um zu überprüfen, ob der Austausch der sauren Aminosäure D (Aspartat) gegen die ebenfalls saure Aminosäure E (Glutamat) in der C-terminalen Spaltstelle von Blr1649 einen Einfluss auf die Calcium-abhängige Autoprotease-Aktivität von GST-Blr1649 hat, wurde ein Derivat des GST-Blr1649 hergestellt, in welchem die Aminosäure $D_{376}$ gegen E ausgetauscht war (GST-Blr1649 $D_{376}E$).

Als Positivkontrolle wurde GST-Blr1649 verwendet. Außerdem wurden als Negativkontrollen GST-Blr1649-Derivate erzeugt, in denen entweder das $D_{164}$ alleine (GST-Blr1649 $D_{164}$A) oder sowohl das $D_{164}$ als auch das $D_{376}$ gegen A (GST-Blr1649 $D_{\frac{164}{376}}$ A) ausgetauscht waren.

Um zu ermitteln, welchen Einfluss die verschiedenen Aminosäureänderungen haben, wurden die Derivate angereichert und mit $CaCl_2$ behandelt. Wie man in Figur 7 sieht, zeigt GST-Blr1649 bei Behandlung mit $CaCl_2$ die erwartete Fragmentierung (Pfeile, Spur WT(+)).

Durch den Aminosäureaustausch $D_{164}$A wurde die erste Spaltstelle eliminiert und es sollten zwei Fragmente mit Größen von 15 kDa und 64 kDa entstehen. Die Bande des Volllängenproteins verschwindet. Die 64 kDa-Bande ist deutlich zu erkennen (Pfeil, Spur D164A (+)). Aufgrund der starken Nebenbanden kann keine genaue Zuordnung der kleineren Fragmentbande getroffen werden.

Durch die Aminosäureaustausch $D_{164}$A und $D_{376}$A wurde die Fragmentierung unterbunden. Das Bandenmuster der mit $CaCl_2$ behandelten Probe besitzt keinen Unterschied zum Ansatz ohne $CaCl_2$.

Bei der Proteinvariante mit dem Aminosäureaustausch $D_{376}$E wurde die Spaltstelle nicht eliminiert. Trotz der veränderten Aminosäure wird eine Calcium-abhängige Autoprotease-Aktivität beobachtet. Es tritt die Fragmentierung wie im Wildtyp-Protein auf, wobei allerdings nur die 42 kDa-Bande deutlich zu erkennen ist.

Blr1806 kann nach einem $D_{147}$E-Austausch ebenfalls gespalten werden (ohne Abbildung).

**Ausführungsbeispiel 3) Untersuchung der Auswirkungen von Metallionen auf Blr1806 mittels SDS-PAGE**

Die Untersuchungen wurden mit nativem Blr1806 durchgeführt. Um zu nativem Blr1806 zu gelangen, wird das GST-Blr1806 vor der Elution auf der Sepharose-Säule mit Thrombin gespalten. Dazu werden zu 940 µl Aufschluss-Puffer 60 µl der Thrombinstammlösung (entspricht 60 Units) gegeben. Die so erhaltene Lösung wurde auf die Säule gegeben. Die Säule wurde verschlossen und für 12 h bei 6°C inkubiert. Anschließend erfolgte die Inkubation für 1 h bei RT. Die Elution erfolgte mit 5 ml Aufschluss-Puffer bei 6°C. Der Säulendurchfluss, welcher den abgetrennten Blr1806-Anteil des Fusionsproteins und Thrombin enthielt, wurde in 500 µl-Fraktionen gesammelt. Diese wurden bis zu maximal einer Woche auf Eis gelagert.

Das Verhalten von Blr1806 nach der Inkubation mit den Salzlösungen zweiwertiger Metalle wurde auch im SDS-PA-Gel untersucht. Dabei wurden den Proteinen vor dem Auftragen auf das Gel die Metallionen durch Zugabe des Chelators EDTA entzogen (Figuren 8 und 9).

Von verschiedenen Calcium-abhängigen Proteasen ist bekannt, dass andere zweiwertige Metallionen ihre Aktivität inhibieren können (Morrissey, 2000). Dies wurde für $Zn^{2+}$ getestet (Figur 8, Spuren F und G).

Um den Einfluss der $Ca^{2+}$-Konzentration genauer zu analysieren, wurden drei verschiedene $Ca^{2+}$-Konzentrationen getestet (Figur 8, Spuren B bis D). Um die Trennschärfe zu erhöhen und eventuelle Fragmente mit noch kleinerer Größe detektieren zu können, wurde ein SDS-PA-Gradientengel (4-20%) benutzt.

Wie die Figuren 8 und 9 zeigen, können andere zweiwertige Metallionen ($Cd^{2+}$, $Mg^{2+}$, $Zn^{2+}$, $Cu^{2+}$, $Fe^{2+}$, $Ni^{2+}$, $Co^{2+}$, $Mn^{2+}$) die Proteolyse von Blr1806 nicht auslösen. $Zn^{2+}$-Ionen scheinen den Effekt jedoch inhibieren zu können. Für die anderen Metalle wurde dies noch nicht getestet. Bei der Inkubation (10 min, RT) mit 0,5 mmol/l $Ca^{2+}$ ist noch eine schwache Bande des Volllängenproteins nachweisbar. Bei Konzentrationen von 1 mmol/l und 2 mmol/l ist dies nicht der Fall.

**Ausführungsbeispiel 4) Untersuchung des zeitlichen Verlaufs der $Ca^{2+}$-abhängigen Proteolyse von Blr1806**

Um den Einfluss des Faktors Zeit auf die Proteolyse von Blr1806 zu untersuchen, wurden Ansätze für unterschiedliche Zeitspannen (10 s bis 2 h) bei Raumtemperatur in Puffer mit oder ohne Calcium inkubiert. Wie aus Figur 10 zu erkennen ist, setzt sich die Proteolyse von Blr1806 nicht weiter als zu den bisher schon beobachteten Fragmenten (25 kDa und 14 kDa) fort.

Bei einer sehr kurzen Inkubationszeit (10 s) konnte noch ein zusätzliches Fragment mit einem Molekulargewicht von ca. 37 kDa beobachtet werden. Dieses Fragment stellt ein Zwischenprodukt der Autoproteolyse dar, bei dem nur an einer Spaltstelle in Blr1806 die Bindung aufgelöst wurde.

Um zu überprüfen, wie sich die Reaktionsgeschwindigkeit bei niedrigeren Temperaturen verändert, wurde die Spaltung bei 0 °C durchgeführt. Dazu wurde die gepufferte $Ca^{2+}$-Lösung bei 0 °C vorinkubiert, anschließend Blr1806 zugegeben und 10 min bei 0 °C inkubiert, wobei nach 0, 0,5, 1, 2, 3, 4, 5, 6, 7, 8, 9, 10, 15 und 20 Minuten Aliquots entnommen wurden und anschließend eine SDS-PAGE durchgeführt wurde (siehe Figur 11). Dabei kann nach etwa 9 bis 10 Minuten eine vollständige Spaltung des Proteins beobachtet werden.

**Ausführungsbeispiel 5) Untersuchung zum Einfluss des pH-Werts auf die Ca$^{2+}$-abhängige Proteolyse von Blr1806**

In einem weiteren Versuch wurde der Einfluss des pH-Werts auf die Calcium-abhängige Autoproteolyse von Blr1806 untersucht. Dabei wurde das Protein nach der Reinigung in Na-Acetat-Puffer (pH 4,0-5,4), in MES-Puffer (pH 5,6-6,6), in Tris-Puffer (pH7,0-9,0) sowie in CAPS-Puffer (pH 9,7 und 10,0) verdünnt. Es wurden Ansätze mit und ohne $CaCl_2$ inkubiert. Wie aus Figur 12 ersichtlich wird, hat der pH-Wert wenig Einfluss auf die Autoproteolyse-Fähigkeit von Blr1806 zwischen pH 5,0-8,0. Bei pH 4,0-4,6 sowie bei pH 8,5-10 findet eine teilweise Spaltung statt.

**Ausführungsbeispiel 6) Spaltung von Blr1806 in Anwesenheit von EDTA**

Um zu überprüfen, ob die Spaltung von Blr1806 auch in Anwesenheit von EDTA möglich ist, wurde Blr1806 in TKE$_{10}$-Puffer (enthält 10 mM EDTA) bei 20°C mit definierten Calciumkonzentrationen (**A**: 0,1 mM, **B:** 0,2 mM, **C:** 0,4 mM:, **D:** 0,7 mM, **E:** 1,4 mM, **F:** 2,8 mM, **G:** 5,7 mM, **H:** 11,4 mM, **I:** 22,7 mM) inkubiert. Die Ansätze wurden anschließend einer SDS-PAGE unterzogen (siehe Figur 13). Ab einer Calciumkonzentration von 2,8 mM konnte eine Spaltung detektiert werden.

Folgende Nicht-Patentliteratur wird in der Erfindungsbeschreibung zitiert:

Arnau J., Lauritzen C., Petersen G. E., Pedersen J. (2006) Current strategies for the use of affinity tags and tag removal for the purification of recombinant proteins. Protein Expr Purif. 48:1-13.

Chong S. et al. (1997) Single-column purification of free recombinant proteins using a selfcleavable affinity tag derived from a protein splicing element. Gene. 192:271-281.

Kenig M., Peternel S., Gaberc-Porekar V., Menart V. (2006) Influence of the protein oligomericity on final yield after affinity tag removal in purification of recombinant proteins. J Chromatogr A. 1101:293-306.

Mao H. (2004) A self-cleavable sortase fusion for one-step purification of free recombinant proteins. Protein Expr Purif. 37:253-263.

Morrissey, J. H. (2000). Coagulation factor VIIa. In A. B. Barret, N. D. Rawlings, J. F. Woessner (Eds.), Handbook of proteolytic enzymes (Kapitel 54, 161-3). Academic Press, London, UK.

Pedersen J., Lauritzen C., Madsen M. T., Weis Dahl S. (1999) Removal of N-terminal polyhistidine tags from recombinant proteins using engineered aminopeptidases. Protein Expr Purif. 15:389-400.

Ruan B., Fisher K. E., Alexander P. A., Doroshko V., Bryan P. N. (2004) Engineering subtilisin into a fluoride-triggered processing protease useful for one-step protein purification. Biochemistiy. 43:14539-14546.

Sadilkovä L. et al. (2008) Single-step affinity purification of recombinant proteins using a self-excising module from Neisseria meningitidis FrpC. Protein Sci. 17:1834-1843.

Zhang A., Gonzalez S. M., Cantor E. J., Chong S. (2001) Construction of a mini-intein fusion system to allow both direct monitoring of soluble protein expression and rapid purification of target proteins. Gene. 275:241-252.

**Patentansprüche**

1. Verwendung eines autoproteolytischen Proteinlinkers als Teil eines Fusionsproteins, welches mindestens ein weiteres, zu exprimierendes Protein als Fusionspartner enthält, **dadurch gekennzeichnet, dass** der autoproteolytische Proteinlinker eine DUF 1521-Domäne ausgewählt aus SEQ ID No. 1 bis 8 oder eine homologe Sequenz mit wenigstens 70% Sequenzidentität zu mindestens einer dieser Sequenzen oder eine Teilsequenz davon enthält, wobei eine Teilsequenz die für die autoproteolytische Aktivität benötigten Abschnitte der DUF 1521-Domäne sowie die Spaltstelle umfasst, wobei die autoproteolytische Spaltung des Proteinlinkers durch Zugabe von $Ca^{2+}$-Ionen induziert wird.

2. Fusionsprotein umfassend

   a) mindestens einen autoproteolytischen Proteinlinker wie in Anspruch 1 definiert,
   b) mindestens ein weiteres, zu exprimierendes Protein als Fusionspartner.

3. Fusionsprotein nach Anspruch 2, **dadurch gekennzeichnet, dass** es einen Affinitätstag enthält.

4. Fusionsprotein nach einem der Ansprüche 2 oder 3, **dadurch gekennzeichnet, dass** ein oder der Fusionspartner ein Markerprotein ist.

5. Fusionsprotein nach einem der Ansprüche 2 bis 4, **dadurch gekennzeichnet, dass** das Fusionsprotein eine Signalsequenz enthält.

6. Fusionsprotein nach einem der Ansprüche 2 bis 5 wobei der autoproteolytische Proteinlinker wie in Anspruch 1 definiert selbst als Affinitätstag dient.

7. Gensequenz kodierend für ein Fusionsprotein nach einem der Ansprüche 2 bis 6.

8. Verwendung einer Gensequenz kodierend für einen autoproteolytischen Proteinlinker wie in Anspruch 1 definiert zur Herstellung eines Fusionsproteins, welches mindestens ein weiteres, zu exprimierendes Protein als Fusionspartner enthält

9. Expressionsvektor umfassend

   a) eine Gensequenz kodierend für einen autoproteolytischen Proteinlinker wie in Anspruch 1 definiert,
   b) mindestens eine Klonierungsstelle oder eine Gensequenz kodierend für ein weiteres, zu exprimierendes Protein (Zielprotein).

10. Wirtszelle umfassend eine Gensequenz gemäß Anspruch 7 und/oder einen Expressionsvektor gemäß Anspruch 9.

11. Kit enthaltend einen Expressionsvektor gemäß Anspruch 9 und eine $Ca^{2+}$-Lösung.

12. Verwendung eines autoproteolytischen Proteinlinkers wie in Anspruch 1 definiert oder einer Gensequenz nach Anspruch 7 oder wie in Anspruch 8 definiert, eines Expressionsvektors nach Anspruch 9 oder einer Wirtszelle nach Anspruch 10 oder eines Kits nach Anspruch 11 zur Expression oder Aufreinigung eines Zielproteins.

13. Verfahren zur Herstellung eines Zielproteins, umfassend die folgenden Verfahrensschritte:

   a) Exprimieren eines Fusionsproteins gemäß einem der Ansprüche 2 bis 6, welches das Zielprotein als Fusionspartner umfasst, in einer Wirtszelle, und
   b) Inkontaktbringen des Fusionsproteins mit einer $Ca^{2+}$-Lösung und Induzierung der Spaltung des autokatalytischen Proteinlinkers, wobei das Zielprotein freigesetzt wird.

14. Verfahren zur Herstellung und Aufreinigung eines Zielproteins, umfassend die folgenden Verfahrensschritte:

   a) Exprimieren des Fusionsproteins gemäß einem der Ansprüche 2 bis 6, welches einen Affinitäts-Tag und das Zielprotein als Fusionspartner enthält, in einer Wirtszelle,
   b) Inkontaktbringen des Fusionsproteins mit einem Liganden, der spezifisch an den Affinitätstag bindet, wobei der Ligand an einer Matrix immobilisiert ist, und

c) Inkontaktbringen des an den Liganden gebundenen Fusionsproteins mit einer Ca$^{2+}$-Lösung und Induzierung der Spaltung des autokatalytischen Proteinlinkers, wobei das Zielprotein freigesetzt wird.

**Claims**

1. Use of an autoproteolytic protein linker as part of a fusion protein, which contains at least one further protein to be expressed as a fusion partner, **characterized in that** the autoproteolytic protein linker contains a DUF 1521 domain selected from SEQ ID NOs:1 to 8 or a homologous sequence with at least 70% sequence identity with at least one of these sequences or a partial sequence thereof, wherein a partial sequence comprises the sections of the DUF 1521 domain required for the autoproteolytic activity as well as the cleavage site, wherein the autoproteolytic cleavage of the protein linker is induced by the addition of Ca$^{2+}$ ions.

2. A fusion protein comprising

   a) at least one autoproteolytic protein linker as defined in claim 1,
   b) at least one further protein to be expressed as a fusion partner.

3. The fusion protein according to claim 2, **characterized in that** it contains an affinity tag.

4. The fusion protein according to claim 2 or claim 3, **characterized in that** a or the fusion partner is a marker protein.

5. The fusion protein according to one of claims 2 to 4, **characterized in that** the fusion protein contains a signal sequence.

6. The fusion protein according to one of claims 2 to 5, wherein the autoproteolytic protein linker as defined in claim 1 itself acts as an affinity tag.

7. A gene sequence coding for a fusion protein according to one of claims 2 to 6.

8. Use of a gene sequence coding for an autoproteolytic protein linker as defined in claim 1 for the production of a fusion protein which contains at least one further protein to be expressed, as a fusion partner.

9. An expression vector comprising

   a) a gene sequence coding for an autoproteolytic protein linker as defined in claim 1,
   b) at least one cloning site or a gene sequence coding for a further protein to be expressed (target protein).

10. A host cell comprising a gene sequence according to claim 7 and/or an expression vector according to claim 9.

11. A kit containing an expression vector according to claim 9, and a Ca$^{2+}$ solution.

12. Use of an autoproteolytic protein linker as defined in claim 1 or of a gene sequence according to claim 7 or as defined in claim 8, of an expression vector according to claim 9 or of a host cell according to claim 10, or of a kit according to claim 11, for the expression or purification of a target protein.

13. A method for the production of a target protein, comprising the following steps of the method:

   a) expressing a fusion protein according to one of claims 2 to 6, which comprises the target protein as a fusion partner, in a host cell, and
   b) bringing the fusion protein into contact with a Ca$^{2+}$ solution and inducing cleavage of the autoproteolytic protein linker, whereupon the target protein is released.

14. A method for the production and purification of a target protein, comprising the following steps of the method:

   a) expressing the fusion protein according to one of claims 2 to 6, which contains an affinity tag and the target protein as a fusion partner, in a host cell, and

b) bringing the fusion protein into contact with a ligand which specifically binds to the affinity tag, whereupon the ligand is immobilized on a matrix, and
c) bringing the fusion protein which is bound to the ligand into contact with a $Ca^{2+}$ solution and inducing cleavage of the autoproteolytic protein linker, whereupon the target protein is released.

## Revendications

1. Utilisation d'un lieur protéique autoprotéolytique comme partie d'une protéine de fusion, qui contient au moins une autre protéine à exprimer comme partenaire de fusion, **caractérisé en ce que** le lieu protéique autoprotéolytique sélectionne un domaine DUF 1521 de SEQ ID No 1 à 8 ou contient une séquence homologue avec au moins 70% d'identité de séquence comme au moins une de ces séquences ou une séquence partielle, dans laquelle une séquence partielle comprend les sections du domaine DUF 1521 nécessaires pour l'activité autoprotéolytique ainsi que le site de clivage, dans laquelle le clivage autoprotéolytique du lieur protéique est induit par l'adjonction d'ions $Ca^{2+}$.

2. Protéine de fusion comprenant

   a) au moins un lieur protéique autoprotéolytique comme défini à la revendication 1,
   b) au moins une autre partenaire à exprimer comme partenaire de fusion.

3. Protéine de fusion selon la revendication 2, **caractérisée en ce que** elle contient une étiquette d'affinité.

4. Protéine de fusion selon une des revendications 2 ou 3, **caractérisée en ce que** un des ou le partenaire de fusion est une protéine de marquage.

5. Protéine de fusion selon une des revendications 2 à 4, **caractérisée en ce que** la protéine de fusion contient une séquence de signaux.

6. Protéine de fusion selon une des revendications 2 à 5, dans laquelle le lieur protéique autoprotéolytique sert d'étiquette d'affinité comme défini à la revendication 1.

7. séquence génétique codant une protéine de fusion selon une des revendications 2 à 6.

8. Utilisation d'une séquence génétique codant un lieur protéique autoprotéolytique comme défini à la revendication 1 pour produire une protéine de fusion, qui contient au moins une autre protéine à exprimer comme partenaire de fusion.

9. Vecteur d'expression comprenant :

   a) une séquence génétique codant un lieu protéique autoprotéolytique comme défini à la revendication 1,
   b) au moins un site de clivage ou une séquence génétique codant une autre protéine à exprimer (protéine cible).

10. Cellule hôte comprenant une séquence génétique selon la revendication 7 et/ou un un vecteur d'expression selon la revendication 9.

11. Kit contenant un vecteur d'expression selon la revendication 9 et une solution de $Ca^{2+}$.

12. Utilisation d'un lieur protéique autoprotéolytique comme défini à la revendication 1 ou une séquence génétique selon la revendication 7 comme défini à la revendication 8, d'un vecteur d'expression selon la revendication 9 ou d'une cellule hôte selon la revendication 10 ou d'un kit selon la revendication 11 pour exprimer ou purifier une protéine cible.

13. Procédé de production d'une protéine cible, comprenant les étapes de procédé suivantes :

   a) exprimer une protéine de fusion selon une des revendications 2 à 6, qui comprend la protéine cible comme partenaire de fusion, dans une cellule hôte et
   b) mettre en contact la protéine de fusion avec une solution de $Ca^{2+}$ et induire le clivage du lieu protéique autoprotéolytique, dans lequel la protéine cible est libérée.

14. Procédé de production et purification d'une protéine cible, comprenant les étapes de procédé suivantes :

a) exprimer la protéine de fusion selon une des revendications 2 à 6, qui contient une étiquette d'affinité et ka protéine cible comme partenaire de fusion, dans une cellule hôte,
b) mettre en contact la protéine de fusion avec un ligand, qui se lie spécifiquement à l'étiquette d'affinité, dans lequel le ligand est immobilisé sur une matrice et
c) mettre en contact la protéine de fusion liée au ligans avec une solution de $Ca^{2+}$ et induire le clivage du lieu protéique autocatalytique, dans lequel la protéine cible est libérée.

```
                                                                          60
1      -WTHEVKDGKATINLGDKYTITANEKDGTWTVRNNQTGH-VTVIHGDPHVDADGDGKDDF
2      -WSHEVRDGKAEIKLGDKYSILVDENDGTVLIRNSQTGK-ITSIKGDPHVDADGDGKVDF
3      -WTHEVKDGKATINLGDKYTITANEKDGTWTVRNNQTGH-VTKIHGDPHVDANGDGKDDF
4      -WSHEVKDGKATINLGDKYTITADEKDGTWTVRNNQTGH-VSRIHGDPHVDANGDGKDDF
5      WSNTQVNDNKSTIDLGN-YKLDLNKKDSSMLLTDKKSGE-TTKVWGDPHIDSN--GTSNM
6      -SASMEGESQASIDLGDGYSLELDERNSEIRIYNASTGE-TTRVWGDPHVDID--GKHAF
7      --SYEPSSGKATLEN-DRYTINIDESSSEIEVIDKQNPEDSFRIYGDPHFDIGNDGDTDF
8      -NSLKVEEGTGKITTPGGYTIEQLG-QFEWRVTGPDGKN--TRVWGDPHVDES--DGGKF
consensus        ..  :   . *.:      .    :  .  .   :  ****.*  .   .  :


                                                                         120
1      DFKKDMTFQLDDGTKITVNNVDY-GNGQ-AISSKLTITNGH-NAIVVEGLGDDKDGKNNL
2      DFKKNMTFQLDDGTKITVDTVDI-GKGK-TMASKLTITNGD-NAMVVEGLGDRFDGKNNL
3      DFKKDMTFQLDDGTKITVNNVDY-GNGE-TISSRLTITNGH-NAMVVEGLGDDKDGKNNL
4      DFKKGMTFQLDDGTKITVDTVNY-GKGK-TISSKLTITNGD-NAMVVEGLGDDKDGKNNL
5      -FNGPLSLNLSDGTKITVGTQ---GKGNVSYADKLTITKGN-DAYLVNGLSEKD--SNPL
6      DFWGTTSFELENGTKITIDTEQYAANPNEYLASKLTITKGD-QAIVVDGISQNQ--IGDL
7      DFKKDMSIELDDGTKLHIHTTPT-SNGE-TLATSLAIEEPDGSGWYIEGIDSDQ--KGDL
8      DFKRDTSFVLGDGTRINCSCKPW-GNGM-TVTGQLDVVYGD-SHVRVTDIDKGKGKVGQV
consensus :*  . :: * :**::      .:      :  * :   . .   : .:..    . :


                                                                         180
1      RVTQSNAGMTLDELTSDGAQTIHES-GQGWVD-GAGRAVNQASIDDGEQGRGPGNYQ--
2      KVTQSNAGRTLDQLTSDGAQTIYEQPGSGWVD-RSGRQVNQEIIDSNENPGTTSD----
3      RVTQSNAGMTLDELTPDGAQTIHES-GQGWVD-GAGGVVNQASIDAGEQGRAPGNYQS-
4      KVTQSNAGRTLDQLTSDGAQTIHET-NQGWVD-NSGRKVTQSVINHNENPDAPPDFKTM
5      TVQHAGNGRQLDAMTPDGYSLVANQNGKGWIDPQTGHAPTAADFKKH-----------
6      SITQSNNGQMLDMLTRDGFVLQENATGAGWRSELTGDIATQADLNLTKVGAIYGPGSE-
7      EVKEYNN------INYSGGTVNDDAALELQVR--DGNYFLNSD---------------
8      TNKGMD----------------------------------------------------
consensus        .. .       . :*. .       .
```

**Fig. 1**

Fig. 2

Fig. 3

Fig. 4

|  | N-terminale Spaltstelle | C-terminale Spaltstelle |
|---|---|---|
|  | -4 -3 -2 -1  +1 +2 +3 +4 +5 | -4 -3 -2 -1  +1 +2 +3 +4 +5 |
| Blr1806$_{wt}$ | ~ I H G D▼P H V D A ~ | ~ I K G D▼P H V D A ~ |
| Blr1806$_{H145A}$ | ~ I **A** G D▼P H V D A ~ | ~ I K G D▼P H V D A ~ |
| Blr1806$_{D147A,D359A}$ | ~ I H G **A**▼P H V D A ~ | ~ I K G **A**▼P H V D A ~ |
| Blr1806$_{I356A}$ | ~ I H G D▼P H V D A ~ | ~**A** K G D▼P H V D A ~ |
| Blr1806$_{K357A}$ | ~ I H G D▼P H V D A ~ | ~ I **A** G D▼P H V D A ~ |
| Blr1806$_{G358A}$ | ~ I H G D▼P H V D A ~ | ~ I K **A** D▼P H V D A ~ |
| Blr1806$_{D359A}$ | ~ I H G D▼P H V D A ~ | ~ I K G **A**▼P H V D A ~ |
| Blr1806$_{P360A}$ | ~ I H G D▼P H V D A ~ | ~ I K G D▼**A** H V D A ~ |
| Blr1806$_{H361A}$ | ~ I H G D▼P H V D A ~ | ~ I K G D▼P **A** V D A ~ |
| Blr1806$_{V362A}$ | ~ I H G D▼P H V D A ~ | ~ I K G D▼P H **A** D A ~ |
| Blr1806$_{D363A}$ | ~ I H G D▼P H V D A ~ | ~ I K G D▼P H V **A** A ~ |

**Fig. 5**

Fig. 6

Fig. 7

Fig. 8

EP 2 391 643 B1

Fig. 9

Fig. 10

Fig. 11

Fig. 12

Fig. 13

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102009008252

### In der Beschreibung aufgeführte Nicht-Patentliteratur

- **ARNAU J. ; LAURITZEN C. ; PETERSEN G. E. ; PEDERSEN J.** Current strategies for the use of affinity tags and tag removal for the purification of recombinant proteins. *Protein Expr Purif.,* 2006, vol. 48, 1-13
- **CHONG S. et al.** Single-column purification of free recombinant proteins using a selfcleavable affinity tag derived from a protein splicing element. *Gene,* 1997, vol. 192, 271-281
- **KENIG M. ; PETERNEL S. ; GABERC-POREKAR V. ; MENART V.** Influence of the protein oligomericity on final yield after affinity tag removal in purification of recombinant proteins. *J Chromatogr A.,* 2006, vol. 1101, 293-306
- **MAO H.** A self-cleavable sortase fusion for one-step purification of free recombinant proteins. *Protein Expr Purif.,* 2004, vol. 37, 253-263
- Coagulation factor VIIa. **MORRISSEY, J. H.** Handbook of proteolytic enzymes. Academic Press, 2000, vol. 54, 161-3
- **PEDERSEN J. ; LAURITZEN C. ; MADSEN M. T. ; WEIS DAHL S.** Removal of N-terminal polyhistidine tags from recombinant proteins using engineered aminopeptidases. *Protein Expr Purif.,* 1999, vol. 15, 389-400
- **RUAN B. ; FISHER K. E. ; ALEXANDER P. A. ; DOROSHKO V. ; BRYAN P. N.** Engineering subtilisin into a fluoride-triggered processing protease useful for one-step protein purification. *Biochemistiy,* 2004, vol. 43, 14539-14546
- **SADILKOVÄ L. et al.** Single-step affinity purification of recombinant proteins using a self-excising module from Neisseria meningitidis FrpC. *Protein Sci.,* 2008, vol. 17, 1834-1843
- **ZHANG A. ; GONZALEZ S. M. ; CANTOR E. J. ; CHONG S.** Construction of a mini-intein fusion system to allow both direct monitoring of soluble protein expression and rapid purification of target proteins. *Gene,* 2001, vol. 275, 241-252

**SEQUENZPROTOKOLL**

<110> Technische Universität Dresden

<120> Autokatalytischer Proteinlinker und seine Verwendung zur Herstellung von Fusionsproteinen

<130> 00017P0123DEWO

<150> DE 10 2009 008 252
<151> 2009-02-02

<160> 15

<170> PatentIn version 3.5

<210> 1
<211> 170
<212> PRT
<213> Bradyrhizobium japonicum

<400> 1

```
        Trp Thr His Glu Val Lys Asp Gly Lys Ala Thr Ile Asn Leu Gly Asp
        1               5                   10                  15

        Lys Tyr Thr Ile Thr Ala Asn Glu Lys Asp Gly Thr Trp Thr Val Arg
                    20                  25                  30

        Asn Asn Gln Thr Gly His Val Thr Val Ile His Gly Asp Pro His Val
                    35                  40                  45

        Asp Ala Asp Gly Asp Gly Lys Asp Asp Phe Asp Phe Lys Lys Asp Met
                50                  55                  60

        Thr Phe Gln Leu Asp Asp Gly Thr Lys Ile Thr Val Asn Asn Val Asp
        65                  70                  75                  80

        Tyr Gly Asn Gly Gln Ala Ile Ser Ser Lys Leu Thr Ile Thr Asn Gly
                        85                  90                  95

        His Asn Ala Ile Val Val Glu Gly Leu Gly Asp Asp Lys Asp Gly Lys
                    100                 105                 110

        Asn Asn Leu Arg Val Thr Gln Ser Asn Ala Gly Met Thr Leu Asp Glu
                    115                 120                 125

        Leu Thr Ser Asp Gly Ala Gln Thr Ile His Glu Ser Gly Gln Gly Trp
                130                 135                 140

        Val Asp Gly Ala Gly Arg Ala Val Asn Gln Ala Ser Ile Asp Asp Gly
        145                 150                 155                 160

        Glu Gln Gly Arg Gly Pro Gly Asn Tyr Gln
                        165                 170
```

<210> 2
<211> 170
<212> PRT
<213> Bradyrhizobium japonicum

<400> 2

```
Trp Ser His Glu Val Arg Asp Gly Lys Ala Glu Ile Lys Leu Gly Asp
1               5               10              15

Lys Tyr Ser Ile Leu Val Asp Glu Asn Asp Gly Thr Val Leu Ile Arg
            20              25              30

Asn Ser Gln Thr Gly Lys Ile Thr Ser Ile Lys Gly Asp Pro His Val
            35              40              45

Asp Ala Asp Gly Asp Gly Lys Val Asp Phe Asp Phe Lys Lys Asn Met
        50              55              60

Thr Phe Gln Leu Asp Asp Gly Thr Lys Ile Thr Val Asp Thr Val Asp
65              70              75              80

Ile Gly Lys Gly Lys Thr Met Ala Ser Lys Leu Thr Ile Thr Asn Gly
                85              90              95

Asp Asn Ala Met Val Val Glu Gly Leu Gly Asp Arg Phe Asp Gly Lys
            100             105             110

Asn Asn Leu Lys Val Thr Gln Ser Asn Ala Gly Arg Thr Leu Asp Gln
        115             120             125

Leu Thr Ser Asp Gly Ala Gln Thr Ile Tyr Glu Gln Pro Gly Ser Gly
    130             135             140

Trp Val Asp Arg Ser Gly Arg Gln Val Asn Gln Glu Ile Ile Asp Ser
145             150             155             160

Asn Glu Asn Pro Gly Thr Thr Ser Asp Ala
                165             170
```

<210> 3
<211> 170
<212> PRT
<213> Bradyrhizobium japonicum

<400> 3

```
Trp Thr His Glu Val Lys Asp Gly Lys Ala Thr Ile Asn Leu Gly Asp
1               5               10              15

Lys Tyr Thr Ile Thr Ala Asn Glu Lys Asp Gly Thr Trp Thr Val Arg
```

```
                    20                        25                        30


        Asn Asn Gln Thr Gly His Val Thr Lys Ile His Gly Asp Pro His Val
                    35                  40                  45


        Asp Ala Asn Gly Asp Gly Lys Asp Asp Phe Asp Phe Lys Lys Asp Met
                    50                  55                  60


        Thr Phe Gln Leu Asp Asp Gly Thr Lys Ile Thr Val Asn Asn Val Asp
        65                  70                  75                  80


        Tyr Gly Asn Gly Glu Thr Ile Ser Ser Arg Leu Thr Ile Thr Asn Gly
                    85                  90                  95


        His Asn Ala Met Val Val Glu Gly Leu Gly Asp Asp Lys Asp Gly Lys
                    100                 105                 110


        Asn Asn Leu Arg Val Thr Gln Ser Asn Ala Gly Met Thr Leu Asp Glu
                    115                 120                 125


        Leu Thr Pro Asp Gly Ala Gln Thr Ile His Glu Ser Gly Gln Gly Trp
                    130                 135                 140


        Val Asp Gly Ala Gly Gly Val Val Asn Gln Ala Ser Ile Asp Ala Gly
        145                 150                 155                 160


        Glu Gln Gly Arg Ala Pro Gly Asn Tyr Gln
                    165                 170
```

<210> 4
<211> 170
<212> PRT
<213> Bradyrhizobium japonicum

<400> 4

Trp Ser His Glu Val Lys Asp Gly Lys Ala Thr Ile Asn Leu Gly Asp
1           5           10          15

Lys Tyr Thr Ile Thr Ala Asp Glu Lys Asp Gly Thr Trp Thr Val Arg
        20          25          30

Asn Asn Gln Thr Gly His Val Ser Arg Ile His Gly Asp Pro His Val
        35          40          45

Asp Ala Asn Gly Asp Gly Lys Asp Asp Phe Asp Phe Lys Lys Gly Met
    50          55          60

Thr Phe Gln Leu Asp Asp Gly Thr Lys Ile Thr Val Asp Thr Val Asn
65          70          75          80

Tyr Gly Lys Gly Lys Thr Ile Ser Ser Lys Leu Thr Ile Thr Asn Gly
            85          90          95

Asp Asn Ala Met Val Val Glu Gly Leu Gly Asp Asp Lys Asp Gly Lys
        100         105         110

Asn Asn Leu Lys Val Thr Gln Ser Asn Ala Gly Arg Thr Leu Asp Gln
        115         120         125

Leu Thr Ser Asp Gly Ala Gln Thr Ile His Glu Thr Asn Gln Gly Trp
    130         135         140

Val Asp Asn Ser Gly Arg Lys Val Thr Gln Ser Val Ile Asn His Asn
145         150         155         160

Glu Asn Pro Asp Ala Pro Pro Asp Phe Lys
            165         170

<210> 5
<211> 156
<212> PRT
<213> Burkholderia phytofirmans

<400> 5

19

```
Trp Ser Asn Thr Gln Val Asn Asp Asn Lys Ser Thr Ile Asp Leu Gly
1               5               10              15

Asn Tyr Lys Leu Asp Leu Asn Lys Lys Asp Ser Ser Met Leu Leu Thr
            20              25              30

Asp Lys Lys Ser Gly Glu Thr Thr Lys Val Trp Gly Asp Pro His Ile
            35              40              45

Asp Ser Asn Gly Thr Ser Asn Met Phe Asn Gly Pro Leu Ser Leu Asn
    50              55              60

Leu Ser Asp Gly Thr Lys Ile Thr Val Gly Thr Gln Gly Lys Gly Asn
65              70              75              80

Val Ser Tyr Ala Asp Lys Leu Thr Ile Thr Lys Gly Asn Asp Ala Tyr
            85              90              95

Leu Val Asn Gly Leu Ser Glu Lys Asp Ser Asn Pro Leu Thr Val Gln
            100             105             110

His Ala Gly Asn Gly Arg Gln Leu Asp Ala Met Thr Pro Asp Gly Tyr
            115             120             125

Ser Leu Val Ala Asn Gln Asn Gly Lys Gly Trp Ile Asp Pro Gln Thr

            130             135             140

Gly His Ala Pro Thr Ala Ala Asp Phe Lys Lys His
    145             150             155
```

<210> 6
<211> 171
<212> PRT
<213> Sphingomonas sp. S88

<400> 6

```
Ser Ala Ser Met Glu Gly Glu Ser Gln Ala Ser Ile Asp Leu Gly Asp
1               5                   10              15

Gly Tyr Ser Leu Glu Leu Asp Glu Arg Asn Ser Glu Ile Arg Ile Tyr
            20              25              30

Asn Ala Ser Thr Gly Glu Thr Thr Arg Val Trp Gly Asp Pro His Val
        35              40              45

Asp Ile Asp Gly Lys His Ala Phe Asp Phe Trp Gly Thr Thr Ser Phe
    50              55              60

Glu Leu Glu Asn Gly Thr Lys Ile Thr Ile Asp Thr Glu Gln Tyr Ala
65              70              75              80

Ala Asn Pro Asn Glu Tyr Leu Ala Ser Lys Leu Thr Ile Thr Lys Gly
            85              90              95

Asp Gln Ala Ile Val Val Asp Gly Ile Ser Gln Asn Gln Ile Gly Asp
        100             105             110

Leu Ser Ile Thr Gln Ser Asn Asn Gly Gln Met Leu Asp Met Leu Thr
        115             120             125

Arg Asp Gly Phe Val Leu Gln Glu Asn Ala Thr Gly Ala Gly Trp Arg
    130             135             140

Ser Glu Leu Thr Gly Asp Ile Ala Thr Gln Ala Asp Leu Asn Leu Thr
145             150             155             160

Lys Val Gly Ala Ile Tyr Gly Pro Gly Ser Glu
            165             170
```

<210> 7
<211> 148
<212> PRT
<213> Vibrio coralliilyticus BAA450

<400> 7

```
Ser Tyr Glu Pro Ser Ser Gly Lys Ala Thr Leu Glu Asn Asp Arg Tyr
1               5                   10                  15

Thr Ile Asn Ile Asp Glu Ser Ser Ser Glu Ile Glu Val Ile Asp Lys
            20                  25                  30

Gln Asn Pro Glu Asp Ser Phe Arg Ile Tyr Gly Asp Pro His Phe Asp
            35                  40                  45

Ile Gly Asn Asp Gly Asp Thr Asp Phe Asp Phe Lys Lys Asp Met Ser
    50                  55                  60

Ile Glu Leu Asp Asp Gly Thr Lys Leu His Ile His Thr Thr Pro Thr
65                  70                  75                  80

Ser Asn Gly Glu Thr Leu Ala Thr Ser Leu Ala Ile Glu Glu Pro Asp
                85                  90                  95

Gly Ser Gly Trp Tyr Ile Glu Gly Ile Asp Ser Asp Gln Lys Gly Asp
            100                 105                 110

Leu Glu Val Lys Glu Tyr Asn Asn Ile Asn Tyr Ser Gly Gly Thr Val
            115                 120                 125

Asn Asp Asp Ala Ala Leu Glu Leu Gln Val Arg Asp Gly Asn Tyr Phe
    130                 135                 140

Leu Asn Ser Asp
145
```

<210> 8
<211> 117
<212> PRT
<213> Myxococcus xanthus DK1622

<400> 8

```
Asn Ser Leu Lys Val Glu Glu Gly Thr Gly Lys Ile Thr Thr Pro Gly
1               5                   10              15

Gly Tyr Thr Ile Glu Gln Leu Gly Gln Phe Glu Trp Arg Val Thr Gly
            20                  25              30

Pro Asp Gly Lys Asn Thr Arg Val Trp Gly Asp Pro His Val Asp Glu
        35                  40              45

Ser Asp Gly Gly Lys Phe Asp Phe Lys Arg Asp Thr Ser Phe Val Leu
        50              55                  60

Gly Asp Gly Thr Arg Ile Asn Cys Ser Cys Lys Pro Trp Gly Asn Gly
65                  70                  75                  80

Met Thr Val Thr Gly Gln Leu Asp Val Val Tyr Gly Asp Ser His Val
                85                  90                  95

Arg Val Thr Asp Ile Asp Lys Gly Lys Gly Lys Val Gly Gln Val Thr
            100                 105                 110

Asn Lys Gly Met Asp
            115
```

<210> 9
<211> 15
<212> PRT
<213> Unknown

<220>
<223> EF-Hand like motif

<220>
<221> misc_feature
<222> (1)..(1)
<223> Xaa can be Val, Ile or Phe

<220>
<221> misc_feature
<222> (3)..(3)
<223> Xaa can be Ala, Ser, Ile or Glu

<220>
<221> misc_feature
<222> (4)..(4)
<223> Xaa can be Asp, Asn, Gly or Ser

<220>
<221> misc_feature
<222> (5)..(5)
<223> Xaa can be Gly or Asn

<220>

<220>
<221> misc_feature
<222> (7)..(7)
<223> Xaa can be Gly or Asp

<220>
<221> misc_feature
<222> (8)..(8)
<223> Xaa can be Lys, Thr, Asp or Gly

<220>
<221> misc_feature
<222> (9)..(9)
<223> Xaa can be Asp, Val, Ser, His, Thr or Gly

<220>
<221> misc_feature
<222> (10)..(10)
<223> Xaa can be Asp, Asn, Ala or Lys

<220>
<221> misc_feature
<222> (11)..(11)
<223> Xaa can be Phe or Met

<220>
<221> misc_feature
<222> (14)..(14)
<223> Xaa can be Lys, Asn or Trp

<220>
<221> misc_feature
<222> (15)..(15)
<223> Xaa can be Lys, Gly or Arg

<400> 9

```
            Xaa Asp Xaa Xaa Xaa Asp Xaa Xaa Xaa Xaa Xaa Asp Phe Xaa Xaa
            1               5                   10              15
```

<210> 10
<211> 12
<212> PRT
<213> Unknown

<220>
<223> Consensus Sequence

<220>
<221> misc_feature
<222> (1)..(1)
<223> Xaa can be Thr or Ser

<220>
<221> misc_feature
<222> (2)..(2)
<223> Xaa can be Phe, Leu or Ile

<220>

<220>
<221> misc_feature
<222> (3)..(3)
<223> Xaa can be Gln, Asn, Glu or Val

<220>
<221> misc_feature
<222> (5)..(5)
<223> Xaa can be Asp, Ser, Glu or Gly

<220>
<221> misc_feature
<222> (6)..(6)
<223> Xaa can be Asp or Asn

<220>
<221> misc_feature
<222> (9)..(9)
<223> Xaa can be Lys or Arg

<220>
<221> misc_feature
<222> (10)..(10)
<223> Xaa can be Ile or Leu

<220>
<221> misc_feature
<222> (11)..(11)
<223> Xaa can be Thr, His or Asn

<220>
<221> misc_feature
<222> (12)..(12)
<223> Xaa can be Val, Ile or Cys

<400> 10

```
Xaa Xaa Xaa Leu Xaa Xaa Gly Thr Xaa Xaa Xaa Xaa
1               5               10
```

<210> 11
<211> 5
<212> PRT
<213> Unknown

<220>
<223> Consensus sequence

<220>
<221> misc_feature
<222> (1)..(1)
<223> Xaa can be Lys, Arg, Ser or Gln

<220>
<221> misc_feature
<222> (3)..(3)
<223> Xaa can be Thr, Ala or Asp

<220>

<221> misc_feature
<222> (4)..(4)
<223> Xaa can be Ile or Val

<220>
<221> misc_feature
<222> (5)..(5)
<223> Xaa can be Thr, Glu or Val

<400> 11

```
Xaa Leu Xaa Xaa Xaa
1                 5
```

<210> 12
<211> 5
<212> PRT
<213> Unknown

<220>
<223> Consensus Sequence

<220>
<221> misc_feature
<222> (1)..(1)
<223> Xaa can be Val or Ile

<220>
<221> misc_feature
<222> (2)..(2)
<223> Xaa can be Glu, Asn, Asp or Thr

<220>
<221> misc_feature
<222> (3)..(3)
<223> Xaa can be Gly or Asp

<220>
<221> misc_feature
<222> (4)..(4)
<223> Xaa can be Leu or Ile

<220>
<221> misc_feature
<222> (5)..(5)
<223> Xaa can be Gly, Ser or Asp

<400> 12

```
Xaa Xaa Xaa Xaa Xaa
1                 5
```

<210> 13
<211> 6
<212> PRT
<213> Unknown

<220>

<223> Consensus sequence

<220>
<221> misc_feature
<222> (1)..(1)
<223> Xaa can be Thr or Asn

<220>
<221> misc_feature
<222> (2)..(2)
<223> Xaa can be Ser, Pro, Arg or Tyr

<220>
<221> misc_feature
<222> (3)..(3)
<223> Xaa can be Asp or Ser

<220>
<221> misc_feature
<222> (5)..(5)
<223> Xaa can be Ala, Tyr, Phe or Gly

<220>
<221> misc_feature
<222> (6)..(6)
<223> Xaa can be Gln, Ser, Val or Thr

<400> 13

```
Xaa Xaa Xaa Gly Xaa Xaa
1                   5
```

<210> 14
<211> 713
<212> PRT
<213> Artificial Sequence

<220>
<223> GST-Blr1806-Fusionsprotein

<400> 14

```
Met Ser Pro Ile Leu Gly Tyr Trp Lys Ile Lys Gly Leu Val Gln Pro
1               5               10              15

Thr Arg Leu Leu Leu Glu Tyr Leu Glu Glu Lys Tyr Glu Glu His Leu
        20              25              30

Tyr Glu Arg Asp Glu Gly Asp Lys Trp Arg Asn Lys Lys Phe Glu Leu
        35              40              45

Gly Leu Glu Phe Pro Asn Leu Pro Tyr Tyr Ile Asp Gly Asp Val Lys
        50              55              60

Leu Thr Gln Ser Met Ala Ile Ile Arg Tyr Ile Ala Asp Lys His Asn
65              70              75              80

Met Leu Gly Gly Cys Pro Lys Glu Arg Ala Glu Ile Ser Met Leu Glu
                85              90              95

Gly Ala Val Leu Asp Ile Arg Tyr Gly Val Ser Arg Ile Ala Tyr Ser
        100             105             110

Lys Asp Phe Glu Thr Leu Lys Val Asp Phe Leu Ser Lys Leu Pro Glu
        115             120             125

Met Leu Lys Met Phe Glu Asp Arg Leu Cys His Lys Thr Tyr Leu Asn
    130             135             140

Gly Asp His Val Thr His Pro Asp Phe Met Leu Tyr Asp Ala Leu Asp
145             150             155             160

Val Val Leu Tyr Met Asp Pro Met Cys Leu Asp Ala Phe Pro Lys Leu
            165             170             175

Val Cys Phe Lys Lys Arg Ile Glu Ala Ile Pro Gln Ile Asp Lys Tyr
        180             185             190

Leu Lys Ser Ser Lys Tyr Ile Ala Trp Pro Leu Gln Gly Trp Gln Ala
        195             200             205

Thr Phe Gly Gly Gly Asp His Pro Pro Lys Ser Asp Leu Val Pro Arg
    210             215             220

Gly Ser Pro Asn Ser Met Gln Tyr Leu Pro Val Ala Gly Leu Pro Val
```

|     | 225 |     |     |     |     | 230 |     |     |     |     | 235 |     |     |     |     | 240 |
|-----|-----|-----|-----|-----|-----|-----|-----|-----|-----|-----|-----|-----|-----|-----|-----|-----|

Val Gly Ala Pro Asp Ser Met Asn Gly Val Ala Pro Glu Gly Ala Val
                245                 250                 255

Val Thr Pro Thr Phe Asn Ala Met Leu Gly Gln Tyr Ala Pro Ala Ser
            260                 265                 270

Tyr Gln Tyr Leu Pro Val Ala Ser Pro Ala Leu Val Gly Thr Ala Val
            275                 280                 285

Gly Ser Val Val Pro Val Val Val Ala Pro Ala Val Val Thr Thr Gln
        290                 295                 300

Ala Tyr Met Met Ala Pro Pro Pro Ser Thr Met Leu Met Ser Gln Leu
305                 310                 315                 320

Asn Gln Asn Ala Glu Pro Pro Val Asp Pro Val Trp Thr His Glu Val
                325                 330                 335

Lys Asp Gly Lys Ala Thr Ile Asn Leu Gly Asp Lys Tyr Thr Ile Thr
                340                 345                 350

Ala Asn Glu Lys Asp Gly Thr Trp Thr Val Arg Asn Asn Gln Thr Gly
            355                 360                 365

His Val Thr Val Ile His Gly Asp Pro His Val Asp Ala Asp Gly Asp
    370                 375                 380

Gly Lys Asp Asp Phe Asp Phe Lys Lys Asp Met Thr Phe Gln Leu Asp
385                 390                 395                 400

Asp Gly Thr Lys Ile Thr Val Asn Asn Val Asp Tyr Gly Asn Gly Gln
            405                 410                 415

Ala Ile Ser Ser Lys Leu Thr Ile Thr Asn Gly His Asn Ala Ile Val
            420                 425                 430

Val Glu Gly Leu Gly Asp Asp Lys Asp Gly Lys Asn Asn Leu Arg Val
        435                 440                 445

Thr Gln Ser Asn Ala Gly Met Thr Leu Asp Glu Leu Thr Ser Asp Gly
    450                 455                 460

Ala Gln Thr Ile His Glu Ser Gly Gln Gly Trp Val Asp Gly Ala Gly
465                 470                 475                 480

Arg Ala Val Asn Gln Ala Ser Ile Asp Asp Gly Glu Gln Gly Arg Gly
                485                 490                 495

```
Pro Gly Asn Tyr Gln Tyr Ala Ser Gly Ala Pro Thr Ser Thr Ala Pro
            500                 505                 510

Val Phe Phe Val Pro Pro Pro Pro Pro Leu Ala Met Val Pro Val
            515                 520                 525

Ala Ala Ala Gln Val Pro Thr Gln Ser Ser Gln Pro Ala Pro Val Trp
    530                 535                 540

Ser His Glu Val Arg Asp Gly Lys Ala Glu Ile Lys Leu Gly Asp Lys
545                 550                 555                 560

Tyr Ser Ile Leu Val Asp Glu Asn Asp Gly Thr Val Leu Ile Arg Asn
                565                 570                 575

Ser Gln Thr Gly Lys Ile Thr Ser Ile Lys Gly Asp Pro His Val Asp
            580                 585                 590

Ala Asp Gly Asp Gly Lys Val Asp Phe Asp Phe Lys Lys Asn Met Thr
            595                 600                 605

Phe Gln Leu Asp Asp Gly Thr Lys Ile Thr Val Asp Thr Val Asp Ile
    610                 615                 620

Gly Lys Gly Lys Thr Met Ala Ser Lys Leu Thr Ile Thr Asn Gly Asp
625                 630                 635                 640

Asn Ala Met Val Val Glu Gly Leu Gly Asp Arg Phe Asp Gly Lys Asn
                645                 650                 655

Asn Leu Lys Val Thr Gln Ser Asn Ala Gly Arg Thr Leu Asp Gln Leu
            660                 665                 670

Thr Ser Asp Gly Ala Gln Thr Ile Tyr Glu Gln Pro Gly Ser Gly Trp
    675                 680                 685

Val Asp Arg Ser Gly Arg Gln Val Asn Gln Glu Ile Ile Asp Ser Asn
    690                 695                 700

Glu Asn Pro Gly Thr Thr Ser Asp Ala
705                 710
```

<210> 15
<211> 740
<212> PRT
<213> Artificial Sequence

<220>

30

<223> GST-Blr1649-Fusionsprotein

<400> 15

```
Met Ser Pro Ile Leu Gly Tyr Trp Lys Ile Lys Gly Leu Val Gln Pro
1               5               10              15

Thr Arg Leu Leu Leu Glu Tyr Leu Glu Glu Lys Tyr Glu Glu His Leu
        20              25              30

Tyr Glu Arg Asp Glu Gly Asp Lys Trp Arg Asn Lys Lys Phe Glu Leu
        35              40              45

Gly Leu Glu Phe Pro Asn Leu Pro Tyr Tyr Ile Asp Gly Asp Val Lys
        50              55              60

Leu Thr Gln Ser Met Ala Ile Ile Arg Tyr Ile Ala Asp Lys His Asn
65              70              75              80

Met Leu Gly Gly Cys Pro Lys Glu Arg Ala Glu Ile Ser Met Leu Glu
                85              90              95

Gly Ala Val Leu Asp Ile Arg Tyr Gly Val Ser Arg Ile Ala Tyr Ser
        100             105             110

Lys Asp Phe Glu Thr Leu Lys Val Asp Phe Leu Ser Lys Leu Pro Glu
        115             120             125

Met Leu Lys Met Phe Glu Asp Arg Leu Cys His Lys Thr Tyr Leu Asn
    130             135             140

Gly Asp His Val Thr His Pro Asp Phe Met Leu Tyr Asp Ala Leu Asp
145             150             155             160

Val Val Leu Tyr Met Asp Pro Met Cys Leu Asp Ala Phe Pro Lys Leu
            165             170             175

Val Cys Phe Lys Lys Arg Ile Glu Ala Ile Pro Gln Ile Asp Lys Tyr
        180             185             190

Leu Lys Ser Ser Lys Tyr Ile Ala Trp Pro Leu Gln Gly Trp Gln Ala
        195             200             205

Thr Phe Gly Gly Gly Asp His Pro Pro Lys Ser Asp Leu Val Pro Arg
        210             215             220

Gly Ser Pro Asn Ser Ala Tyr Leu Pro Val Ala Gly Leu Pro Val Val
225             230             235             240

Gly Ala Leu Asp Ser Thr Val Asn Gly Val Gly Ser Glu Gly Val Val
            245             250             255
```

```
Ala Ser Pro Ala Phe Ser Thr Leu Leu Gly Gln Tyr Val Thr Pro Gly
        260             265             270

Ala Tyr Gln Tyr Leu Pro Ala Gly Ala Ala Val Val Val Ala Pro Val
        275             280             285

Val Val Pro Ala Gly Gly Val Thr Thr Ala Val Val Ala Pro Ala Val
        290             295             300

Val Thr Pro Val Val Ala Ala Pro Val Tyr Met Met Ala Pro Pro Pro
305             310             315             320

Pro Phe Met Leu Leu Ser Gln Gln Ser Ser Gly Ser Glu Pro Ser Val
        325             330             335

Asp Pro Val Trp Thr His Glu Val Lys Asp Gly Lys Ala Thr Ile Asn
        340             345             350

Leu Gly Asp Lys Tyr Thr Ile Thr Ala Asn Glu Lys Asp Gly Thr Trp
        355             360             365

Thr Val Arg Asn Asn Gln Thr Gly His Val Thr Lys Ile His Gly Asp
        370             375             380

Pro His Val Asp Ala Asn Gly Asp Gly Lys Asp Asp Phe Asp Phe Lys
385             390             395             400

Lys Asp Met Thr Phe Gln Leu Asp Asp Gly Thr Lys Ile Thr Val Asn
            405             410             415

Asn Val Asp Tyr Gly Asn Gly Glu Thr Ile Ser Ser Arg Leu Thr Ile
        420             425             430

Thr Asn Gly His Asn Ala Met Val Val Glu Gly Leu Gly Asp Asp Lys
        435             440             445

Asp Gly Lys Asn Asn Leu Arg Val Thr Gln Ser Asn Ala Gly Met Thr
        450             455             460

Leu Asp Glu Leu Thr Pro Asp Gly Ala Gln Thr Ile His Glu Ser Gly
465             470             475             480

Gln Gly Trp Val Asp Gly Ala Gly Gly Val Val Asn Gln Ala Ser Ile
            485             490             495

Asp Ala Gly Glu Gln Gly Arg Ala Pro Gly Asn Tyr Gln Ser Ala Ser
        500             505             510
```

```
Ala Thr Pro Ala Ser Ala Thr Pro Val Phe Phe Val Pro Pro Pro
        515                 520             525

Pro Pro Leu Ala Met Val Pro Phe Ala Val Ala Gln Thr Pro Ala Pro
        530                 535             540

Ser Ser Gln Pro Ala Pro Val Trp Ser His Glu Val Lys Asp Gly Lys
545             550             555                 560

Ala Thr Ile Asn Leu Gly Asp Lys Tyr Thr Ile Thr Ala Asp Glu Lys
            565                 570                 575

Asp Gly Thr Trp Thr Val Arg Asn Asn Gln Thr Gly His Val Ser Arg
            580                 585             590

Ile His Gly Asp Pro His Val Asp Ala Asn Gly Asp Gly Lys Asp Asp
        595                 600             605

Phe Asp Phe Lys Lys Gly Met Thr Phe Gln Leu Asp Asp Gly Thr Lys
    610                 615                 620

Ile Thr Val Asp Thr Val Asn Tyr Gly Lys Gly Lys Thr Ile Ser Ser
625             630                 635                 640

Lys Leu Thr Ile Thr Asn Gly Asp Asn Ala Met Val Val Glu Gly Leu
            645                 650                 655

Gly Asp Asp Lys Asp Gly Lys Asn Asn Leu Lys Val Thr Gln Ser Asn
            660                 665                 670

Ala Gly Arg Thr Leu Asp Gln Leu Thr Ser Asp Gly Ala Gln Thr Ile
            675                 680                 685

His Glu Thr Asn Gln Gly Trp Val Asp Asn Ser Gly Arg Lys Val Thr
        690                 695                 700

Gln Ser Val Ile Asn His Asn Glu Asn Pro Asp Ala Pro Pro Asp Phe
705                 710                 715                 720

Lys Thr Met Met Arg Glu Ala Met Phe Arg Arg Ile Phe Gly Arg Asp
                725                 730                 735

Ala Gln Pro Ala
            740
```